# EUROPEAN PATENT SPECIFICATION

(11) **EP 0 589 047 B1**
(45) Date of publication and mention of the grant of the patent: **09.06.1999**
(21) Application number: 93905631.3
(22) Date of filing: 10.03.1993
(51) Int. Cl.: A01N 65/00, A61K 7/00

(54) **METHOD OF TREATING INFECTIOUS DISEASE, METHOD OF PREVENTING PUTREFACTION OF COSMETIC, AND ANTIBACTERIAL/ANTIFUNGAL AGENT AND COSMETIC**
VERFAHREN ZUR BEHANDLUNG INFEKTIÖSER KRANKHEITEN, VERFAHREN ZUR VERHINDERUNG DES VERDERBS VON KOSMETIKA, ANTIBAKTERIELLE UND SCHIMMELHEMMENDE MITTEL SOWIE KOSMETIKA
PROCEDE DE TRAITEMENT DE MALADIES INFECTIEUSES, PROCEDE PERMETTANT D'EMPECHER LA PUTREFACTION DES PRODUITS COSMETIQUES, ET PRODUIT COSMETIQUE ET AGENT ANTIBACTERIENS/ANTIFONGIQUES

(30) Priority: 13.03.1992 JP 5507492; 11.08.1992 JP 21384192
(43) Date of publication of application: 30.03.1994
(73) Proprietor: OTSUKA PHARMACEUTICAL CO., LTD., Chiyoda-ku, Tokyo 101-0048 (JP)
(72) Inventor: OTSU, Yoshiro, Minoo-shi, Osaka 562 (JP); ARIMA, Yaeno, Kobe-shi, Hyogo 654-01 (JP); NAKAI, Yoriko, Hikami-gun, Hyogo 669-33 (JP)
(74) Representative: Hansen, Bernd, Dr. Dipl.-Chem.
(86) International application number: JP9300297
(87) International publication number: WO9317559

(56) References cited:
- JP-A- 2 157 201
- JP-A- 56 008 309
- JP-A- 62 093 215
- PATENT ABSTRACTS OF JAPAN vol. 12, no. 501 (C-556) (3348) 27 December 1988 & JP-A-63 211 218 (NIPPON ZEORA K.K.) 2 September 1988

## Description

### TECHNICAL FIELD

This invention relates to the use of specific agents in the preparation of a medicament effective against infectious diseases caused by bacteria or fungi, to a method of preserving cosmetics, to antibacterial and/or antifungal agents, and to cosmetics.

### BACKGROUND ART

Zinc oxide is generally applied to affected sites of the skin in eczema, dermatitis, abrasion, burn or the like as a local astringent, desiccant and protectant and for relief of stimulations and absorption of secretion. This substance is used in various dosage forms, such as powder, solution, oil, lotion, liniment, ointment, paste, plaster and so on. Particularly, the ointment is used most generally.

Referring to the ointment, the Japanese Pharmacopoeia lists Zinc Oxide Ointment. However, this ointment has the drawback that because it does not contain any bactericide or preservative, the skin lesions become more susceptible to the infection of bacteria and fungi when it is repeatedly used over a long time.

Zinc oxide ointments supplemented with ichthammol, acrinol, boric acid or the like are also known. However, these ointments also have various drawbacks. Ichthammol, which is incorporated for reducing inflammation or relieving itching, has a peculiar odor and a brownish black color, so that zinc oxide ointments containing ichthammol have been delisted from the new Japanese Pharmacopeia (12th edition). Acrinol, which is incorporated for purposes of local sterilization or disinfection, may sometimes induce symptoms of irritation and has the drawback that it readily undergoes discoloration upon exposure to light and stains clothes yellow. Boric acid, which is incorporated for the purpose of sterilization, has only a weak inhibitory effect on bacterial growth and, when continuously used over a long period of time, it is absorbed through the skin and may cause poisoning, such as nausea and vomiting or diarrhea. Therefore, the use of this substance is rigorouly controlled.

JP-A-56-8309 discloses in example 1 a lotion which contains, besides other ingredients, hinokitiol and zinc phenolsulphonate.

US-5,219,847 describes in example 16 a hair tonic which contains, besides other ingredients, zinc dipicolinate and hinokitiol.

JP-A-63-211218 discloses an oral cavity composition which shows an improved storage stability. This is achieved by adding edetic acid or a salt thereof and a carboxylic acid based-amphoteric surfactant to hinokitiol-containing oral cavity compositions. Among many examples for the salts of edetic acid, the disodium zinc salt is mentioned.

Furthermore, the above-mentioned various zinc oxide ointments containing antimicrobial agents are not sufficiently safe or low in irritation. Thus, they can hardly be applied to patients sensitive to irritation.

### DISCLOSURE OF INVENTION

In view of the above state of the art, we made intensive investigations in an attempt to develop an antibacterial and antigungal agent having excellent antibacterial and antifungal activities. As a result, we found that when a zinc compound, inclusive of zinc oxide and the like, is used in combination with hinokitiol or a salt thereof, markedly improved antibacterial and antifungal activities are obtained. We also found that, due to these excellent antibacterial and antifungal activities, the combination of a zinc compound and hinokitiol or a salt thereof is very useful particularly for treating infectious diseases caused by bacteria or fungi, for preserving cosmetics, and for other purposes. The present invention has been completed based on the above findings.

Thus, the present invention provides the use of a zinc compound and at least one member selected from the group consisting of hinokitiol and salts thereof for preparing a medicament effective in the treatment of an infectious disease caused by bacteria or fungi.

The invention further provides a method of preserving a cosmetic which is characterized in that said cosmetic contains an effective amount of a zinc compound and an effective amount of hinokitiol or a salt thereof.

Furthermore, the invention is concerned with an antibacterial and/or antifungal agent which comprises an effective amount of a zinc compound and an effective amount of hinokitiol or a salt thereof under the proviso that the zinc compound is neither zinc dipicolinate nor a zinc salt of edetic acid. The invention is also concerned with a cosmetic which contains a zinc compound and at least one member selected from the group consisting of hinokitiol and salts thereof under the proviso that the zinc compound is not represented by zinc paraphenolsulfonate, zinc phenolsulfonate, or zinc dipicolinate.

In the practice of the invention, the zinc compound to be used is not limited to any particular species but includes a wide variety of per se known zinc compounds, such as, for example, zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paraphenolsulfonate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc phenolsulfonate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc dipicolinate, zinc-glycerol complex, zinc-bishistidine complex, zinc-3,4-dihydroxybenzoic acid complex, zinc nicotinate, zinc-nicotinamide complex, and other zinc complexes and zinc salts. In the practice of the invention, these zinc compounds may be used either singly or in combination as a mixture of two or more.

Examples of the salt of hinokitiol to be used in the practice of the invention are inorganic salts including alkali metal salts such as sodium salt and potassium salt, alkaline earth metal salts such as magnesium salt and other metal salts such as copper salt and zinc salt, and organic salts including alkanolamine salts such as diethanolamine salt, 2-amino-2-ethyl-1,3-propanediol salt and triethanolamine salt, heterocyclic amine salts such as morpholine salt, piperazine salt and piperidine salt, ammonium salt, and basic amino acid salts such as arginine salt, lysine salt and histidine salt. The basic amino acids each may be in the D form or in the L form or in the form of a mixture of these. In the practice of the invention, hinokitiol and salts thereof may be used either singly or two or more of them may be used in combination.

The proportions of the zinc compound and hinokitiol or a salt thereof to be incorporated into the compositions of the invention are not particularly limited provided that the intended effects of the invention can be produced. Generally, however, it is recommended that both are incorporated in a former:latter weight ratio of about 10-99.95:90-0.05, preferably about 50-99.9:50-0.1. Thus, it is recommended to use the zinc compound in an amount of about 10 to 99.95% by weight, preferably about 50 to 99.9% by weight, and to use hinokitiol or a salt thereof in an amount of about 90 to 0.05% by weight, preferably about 50 to 0.1% by weight, based on the total amount of the zinc compound and hinokitiol or the salt thereof.

When the compositions of the invention are to be used as medicaments, the zinc compound and hinokitiol or a salt thereof may be used in a manner such that both of them are contained in a single pharmaceutical preparation. It is also possible to formulate them independently into separate preparations and utilize both of the pharmaceutical preparations. Such preparations are produced by using diluents or excipients that are generally used, including fillers, bulking agents or extenders, binders, humectants, disintegrating agents, surfactants, lubricants and so on. For these pharmaceutical preparations, various forms can be selected depending on the purpose of therapy and, as typical examples thereof, there may be mentioned tablets, pills, powders, solutions, suspensions, emulsions, granules, capsules, suppositories, injections (solutions, suspensions, etc.), oleaginous ointments, emulsion ointments, water-soluble ointments, pastes, plasters, lotions, liniments and other preparations for external use.

When the compositions of the invention are to be used as cosmetics, they can be added as antidandruff agents, bactericides and/or preservatives to the cosmetics for enabling preservation of the cosmetics, for instance. Said cosmetics may be used in various forms including skin care products such as cleansing preparations, creams, milk lotions, makeup creams, oils, packs or the like; makeup cosmetics such as foundations, lipsticks, cheek rouges, eyeliners, mascaras, eye shadows, manicures, face powders or the like; hair care preparations such as hair dressing preparations, hair tonics or the like; bath preparations, whitening preparations, sunscreen preparations, acne treatment preparations, etc. These can be produced by the methods that are conventional in the art.

In producing such cosmetics, various known cosmetic base materials, such as vehicles, binders, lubricants, disintegrating agents, etc., can be used as necessary and, further, various oleaginous materials, such as oils, fats, waxes, hydrocarbons, fatty acids, higher alcohols, ester oils, metal soaps, etc., pharmacologically active materials, such as animal or plant extracts, vitamins, hormones, amino acids, etc., surfactants, colors, dyes, pigments, perfumes, preservatives, bactericides, humectants, thickening agents, anti-oxidants, metal sequestering agents and other already known various components or additives can be used as necessary in a suitable combination.

In using the compositions of the invention as medicaments, the active ingredient concentrations are not particularly limited provided that the compositions have the desired effects or benefits. Generally, however, they recommendably contain a zinc compound and hinokitiol or a salt thereof in a total amount of about 0.001 to 20% by weight, preferably about 0.01 to 15% by weight.

In cases where the compositions of the invention are to be used as cosmetics, the concentration thereof may vary depending on the form and the like, hence cannot be specified in a general manner or are not particularly limited. Generally, however, the compositions recommendably contain a zinc compound and hinokitiol or a salt thereof in a total amount of about 0.0001 to 99.9% by weight, preferably about 0.001 to 30% by weight. The cosmetics mentioned above may be used after further dilution with water, olive oil or some other suitable solvent.

In shaping into the form of tablets, a variety of materials so far well known as carriers or vehicles in this field of art can be used. Thus, for example, use may be made of excipients such as lactose, sucrose, sodium chloride, glucose, urea, starch, calcium carbonate, kaolin, crystalline cellulose, silicic acid, etc., binders such as water, ethanol, propanol, simple syrup, glucose solution, starch solution, gelatin solution, carboxymethylcellulose, shellac, methylcellulose, potassium phosphate, polyvinylpyrrolidone, etc., disintegrating agents such as dried starch, sodium alginate, agar powder, laminaran powder, sodium hydrogen carbonate, calcium carbonate, polyoxyethylenesorbitan fatty acid esters, sodium lauryl sulfate, stearic acid monoglyceride, starch, glucose, etc., disintegration preventing agents such as sucrose, stearin, cacao butter, hydrogenated oils, etc., absorption promoting agents such as quaternary ammonium bases, sodium lauryl sulfate, etc., humectants such as glycerin, starch, etc., adsorbents such as starch, lactose, kaolin, bentonite, colloidal silicic acid, etc., lubricants such as purified talc, stearic acid salts, corn starch, waxes, polyethylene glycol, etc., and so forth. The tablets may further be processed to tablets coated with an ordinary coating film, for example sugar coated tablets, gelatin coated tablets, enteric-film coated tablets, film coated tablets, or double-layered tablets or multilayered tablets.

In shaping into the form of pills, a wide variety of materials so far known as carriers or vehicles in this field of art can be used. Thus, for example, use may be made of excipients such as glucose, lactose, starch, cacao butter, hardened vegetable oils, kaolin, talc, etc., binders such as gum arabic powder, tragacanth powder, gelatin, ethanol, etc., disintegrating agents such as laminaran, agar, etc., and so forth.

In shaping into the form of suppositories, a wide variety of materials so far known as carriers or vehicles in the art can be used. Thus, for example, there may be mentioned polyethylene glycol, cacao butter, higher alcohols, higher alcohol esters, gelatin, semisynthetic glycerides, and so on.

In preparing injections, the solutions, emulsions and suspensions are sterilized and are preferably isotonic with blood. For producing these forms, all diluents conventionally used in this field can be employed. Thus, for instance, water, ethyl alcohol, macrogols, propylene glycol, ethoxylated isostearyl alcohol, polyoxylated isostearyl alcohol, polyoxyethylene sorbitan fatty acid esters and the like can be used. In this case, sodium chloride, glucose or glycerin may be incorporated into the pharmaceutical preparations in an amount sufficient to give isotonic solutions. Convenitional solubilizing agents, buffers, soothing agents and the like may also be added.

In preparing ointments, a wide variety of oleaginous bases so far known in this field can be used. As specific examples, there may be mentioned fats and oils such as peanut oil, sesame oil, soybean oil, safflower oil, avocado oil, sunflower oil, corn oil, rapeseed oil, cottonseed oil, castor oil, camellia oil, coconut oil, olive oil, poppy seed oil, cacao oil, beef tallow, lard, wool oil, etc. (lard being particularly preferred), mineral oils such as vaseline, paraffin, silicone oil, squalane, etc. (white vaseline being particularly preferred), higher fatty acid esters, higher aliphatic alcohols and waxes such as isopropyl myristate, n-butyl myristate, isopropyl linoleate, propyl ricinoleate, isopropyl ricinoleate, isobutyl ricinoleate, heptyl ricinoleate, diethyl sebacate, diisopropyl adipate, cetyl alcohol, stearyl alcohol, bleached beeswax, spermaceti, Japan wax, etc, higher fatty acids such as stearic acid, oleic acid, palmitic acid, etc., mono-, di- and triglyceride mixtures derived from saturated or unsaturated fatty acids containing 12 to 18 carbon atoms (lipophilic glycerin monostearate being particularly preferred), and so on. In the practice of the invention, these bases may be used either singly or in combination as a mixture of two or more.

Conventional additives, for example metal soaps, animal or plant extracts, vitamins, hormones, amino acids or other pharmacologically active substances, surfactants, coloring matters, dyes, pigments, perfumes, ultraviolet absorbers, humectants, thickening agents, antioxidants, metal sequestering agents, pH adjusting agents, etc., may be incorporated, as necessary, into the compositions of the invention.

The compositions of the invention are produced by a conventional method.

The method of administration of the above-mentioned pharmaceutical preparations is not particularly limited but the preparations are administered by a method suitably selected depending on the dosage form, the age, sex and other conditions of the patient, the severity of the disease and other factors. Thus, tablets, pills, solutions, suspensions, emulsions, granules and capsules, for instance, are administered orally. Injections are administered, either as such or in admixture with an ordinary parenteral fluid containing glucose, amino acids or the like, by the intravenous route. If desired, they are administered as such via intramuscular, intradermal, subcutaneous or intraperitoneal route. Suppositories are administered rectally. External preparations are applied to the affected parts.

These pharmaceutical preparations of the present invention are recommendably administered in such an amount that the total amount of the active ingredients, i.e., a zinc compound and hinokitiol or a salt thereof is about 1 to 20 mg/kg body weight per human adult per day, and the preparations are administered in a single dose or two or three divided doses.

The compositions of the invention have excellent antibacterial and antifungal activities and are particularly useful in the treatment of various infectious diseases caused by various bacteria and/or fungi, for example various skin diseases including dermatitis such as atopic dermatitis, nummular dermatitis, autosensitization dermatitis, diaper dermatitis and stasis dermatitis, eczema such as housewives (hand) eczema and dry eczema, pustular psoriasis, burn, redness resulting from burn, rash, miliaria, sore, abrasion, secondary infection through scratching, etc.

In addition, the compositions of the invention are very low in skin irritancy and allergy-inducing potential and therefore can be applied not only to babies, infants, children and other patients with skin diseases, for example patients susceptible to skin irritation, but also in the treatment of skin diseases in mammals other than humans (pet animals such as dogs, cats, etc., domestic animals such as cattle, horses, etc., and so on).

The compositions of the invention possess excellent antibacterial and antifungal activities, are safe and very weak in irritancy, and can be suitably used in babies, infants, children and other patients with skin diseases, patients susceptible to irritation and so forth. The compositions of the invention can also be suitably used in patients with atopic dermatitis or contact dermatitis.

### EXAMPLES

The following production examples and test examples are further illustrative of the present invention. Hereinafter, "%" means "% by weight".

| Production Example 1 (Zinc oxide ointment) | |
|---|---|
| Hinokitiol | 0.5 g |
| Lard | 300.0 g |
| Bleached beeswax | 60.0 g |
| Zinc oxide | 100.0 g |
| White petrolatum | q.s. |
| Total | 1000.0 g |

(1) Hinokitiol was weighed, and a portion of lard was added thereto, and the mixture was heated to about 40°C and stirred to give a homogeneous melt. (2) The remaining portion of lard, bleached beeswax and white petrolatum were dissolved on a water bath and stirred to give a mixture at 80°C. (3) Zinc oxide was weighed into a mortar. Thereto was added portionwise the mixture prepared in the above-mentioned step (2) with stirring to give a homogenous mixture, which was then cooled to about 40°C with stirring. (4) Then, the mixture prepared in the above step (1) was added at about 40°C and stirring was continued until solidification to give the desired zinc oxide ointment.

| Production Example 2 (Zinc oxide ointment) | |
|---|---|
| Hinokitiol | 0.1 g |
| Olive oil | 100.0 g |
| Zinc oxide | 100.0 g |
| Simple ointment (Japanese Pharmacopoeia) | q.s. |
| Total | 1000.0 g |

(1) Hinokitiol was weighed. To this was added a portion of olive oil. The mixture was heated to about 40°C and stirred to give a homogeneous melt. (2) The remaining portion of olive oil and simple ointment (Japanese Pharmacopoeia) were dissolved on a water bath and then stirred to give a mixture at 80°C. (3) Zinc oxide was weighed into a mortar. Thereto was added portionwise with stirring the mixture prepared in the above step (2) to give a homogenous mixture, which was then cooled to about 40°C with stirring. (4) Then, the mixture prepared in the above step (1) was added at about 40°C, followed by stirring well until solidification to give the desired zinc oxide ointment.

| Production Example 3 (Absorptive zinc oxide ointment) | |
|---|---|
| Lipophilic glycerin monostearate | 50.0 g |
| Zinc oxide | 50.0 g |
| White petrolatum | q.s. |
| Hinokitiol sodium salt | 0.05 g |
| Glycerin | 30.0 g |
| Purified water | 50.0 g |
| Total | 1000.0 g |

(1) Hinokitiol sodium salt, glycerin and purified water were heated to about 60°C and stirred to give a homogeneous melt. (2) Lipophilic glycerin monostearate and white petrolatum were dissolved on a water bath and then stirred to give a mixture at 60°C. (3) The mixture prepared in the above step (1) was added portionwise to the mixture prepared in the above step (2) with uniform stirring. The whole mixture was then cooled to about 40°C with stirring. (4) Zinc oxide was weighed into a mortar. Thereto was added portionwise the mixture prepared in the above step (3), followed by thorough stirring until solidification to give the desired zinc oxide ointment.

| Production Example 4 (Vanishing cream) | |
|---|---|
| Stearic acid | 10.0% |
| Paraffin wax (135F) | 2.0% |
| Spermaceti | 2.0% |
| Cetyl alcohol | 2.0% |
| Cetyl isooctanoate | 5.0% |
| Polyoxyethylenesorbitan (20EO) monolaurate | 3.0% |
| Zinc acetate | 0.015% |
| Sodium hydroxide | 0.15% |
| Concentrated glycerin | 5.0% |
| Hinokitiol | 0.02% |
| Perfume | suitable amount |
| Purified water | Balance |
| Total | 100.0% |

(1) Stearic acid, paraffin wax (135F), spermaceti, cetyl alcohol, cetyl isooctanoate and polyoxyethylenesorbitan (20EO) monolaurate were heated to 80-85°C to give a homogeneous melt. (2) Zinc acetate, sodium hydroxide, concentrated glycerin and purified water were heated to 80-85°C and stirred to give a homogeneous mixture. (3) At 80°C, the mixture prepared in the above step (2) was added portionwise to the mixture prepared in the above step (1) and, after uniform emulsification, the whole mixture was cooled to 45°C with stirring. (4) At 45°C, hinokitiol and perfume were added to the mixture prepared in the above step (3), and the mixture was stirred to homogeneity and cooled to room temperature with stirring to give the desired vanishing cream. Said vanishing cream was in the form of a stable emulsion.

| Production Example 5 (Cleansing cream) | |
|---|---|
| Bleached beeswax | 3.0% |
| Liquid paraffin | 30.0% |
| Cetyl alcohol | 2.0% |
| Cetyl isooctanoate | 10.0% |
| Triethanolamine | 0.2% |
| Propylene glycol | 5.0% |
| Zinc acetate | 0.1% |
| Antioxidant | suitable amount |
| Hinokitiol | 0.05% |
| Perfume | suitable amount |
| Purified water | Balance |
| Total | 100.0% |

The above ingredients were admixed for emulsification following the procedure of Production Example 4 to give a stable emulsion.

| Production Example 6 (Milk lotion) | |
|---|---|
| Stearic acid | 3.0% |
| Spermaceti | 3.0% |
| Lipophilic glycerin monostearate | 2.0% |
| Bleached beeswax | 2.0% |
| Saturated fatty acid (C₈-C₁₂) triglyceride | 10.0% |
| L-Arginine | 1.0% |
| Sorbitol | 3.0% |
| Zinc pyrithione | 0.02% |
| Hinokitiol | 0.02% |
| Perfume | 0.1% |
| Purified water | Balance |
| Total | 100.0% |

The above ingredients were admixed for emulsification following the procedure of Production Example 4, whereupon a stable emulsion was obtained.

| Production Example 7 (Lotion) | |
|---|---|
| Ethyl alcohol | 10.0% |
| Polyoxyethylene (9EO) lauryl ether | 2.0% |
| Kankoh SO 201 | 0.001% |
| Perfume | suitable amount |
| Concentrated glycerin | 5.0% |
| 1,3-Butylene glycol | 3.0% |
| Hinokitiolato zinc | 0.05% |
| Hinokitiol | 0.05% |
| Color | suitable amount |
| Purified water | Balance |
| Total | 100.0% |

(1) Polyoxyethylene (9EO) lauryl ether, Kankoh SO 201 and perfume were added to and uniformly dissolved in ethyl alcohol. (2) Concentrated glycerin, 1,3-butylene glycol and hinokitiolato zinc were added to and uniformly dissolved in purified water. (3) At 80°C, the mixture prepared in the above step (2) was added to the mixture prepared in the above step (1) and, after uniform stirring for solubilization, hinokitiol was added and color was further added for coloration to give a lotion.

| Production Example 8 (Zinc oxide ointment) | |
|---|---|
| Lard | 20.95% |
| Bleached beeswax | 7.0% |
| Lipophilic glycerin monostearate | 2.0% |
| White petrolatum | 60.0% |
| Zinc oxide | 10.0% |
| Hinokitiol | 0.05% |
| Total | 100.0% |

(1) Hinokitiol was weighed. To this was added a portion of lard and the mixture was heated to about 40°C and stirred to give a homogeneous melt. (2) The remaining portion of lard, lipophilic glycerin monostearate, bleached beeswax and white petrolatum were dissolved on a water bath and stirred to give a mixture at 80°C. (3) Zinc oxide was weighed into a mortar. To this was added portionwise the mixture prepared in the above step (2) with uniform stirring. The resulting mixture was cooled to about 40°C with stirring. (4) Then, the mixture prepared in the above step (1) was added at about 40°C. The whole mixture was stirred well until solidification to give the desired zinc oxide ointment.

### Test Example 1 (Antibacterial activity test)

The drugs tested were hinokitiol SP (Takasago Perfumery Co., Ltd.; Lot 112108) and zinc oxide (Wako Pure Chemical Industries, Ltd.; Lot 71620). The test bacterial strain used was Staphylococcus epidermidis KPU1 (Staphylococcus epidermidis).

Cells of the above test strain precultured on SCD slant medium (18 to 20 hours, 37°C) were suspended in SCD medium and the suspension was diluted and adjusted to 10⁶ to 10⁷ cells/ml and allowed to stand for several tens of minutes. This cell suspension was added to drug solutions which had been adjusted to predetermined drug concentrations. Then, shaking culture was performed (diluent: SCDLP medium or sterile physiological saline; medium: SCDLP agar medium; incubation temperature: 37°C; incubation period: 24 to 48 hours) and viable cells were counted at timed intervals.

The concentrations of drugs for testing and the method of preparing the drugs were as follows. Thus, a control was prepared by adding 9 ml of the cell suspension to 1 ml of sterile distilled water. Hinokitiol (0.02%) was prepared by diluting an alcohol solution containing hinokitiol at a concentration of 5% with sterile distilled water to give a 0.2% hinokitiol solution and adding 9 ml of the cell suspension to 1 ml of said 0.2% solution. Hinokitiol (0.04%) was prepared by diluting an alcohol solution containing hinokitiol at a concentration of 5% with sterile distilled water to give a 0.4% hinokitiol solution and adding 9 ml of the cell suspension to 1 ml of said 0.4% solution. Zinc oxide (0.1%) was prepared by adding 10 ml of the cell suspension to 0.01 g of zinc oxide (ZnO). Hinokitiol (0.04%) + zinc oxide (0.1%) was prepared by adding 1 ml of the 0.4% hinokitiol solution to 0.01 g of zinc oxide, followed by addition of 9 ml of the cell suspension. Zinc acetate (0.4%) was prepared by adding 10 ml of the cell suspension to 0.04 g of zinc acetate (Zn(CH₃COO)₂·2H₂O). Hinokitiol (0.02%) + zinc acetate (0.4%) was prepared by adding 1 ml of the 0.2% hinokitiol solution to 0.04 g of zinc acetate, followed by addition of 9 ml of the cell suspension.

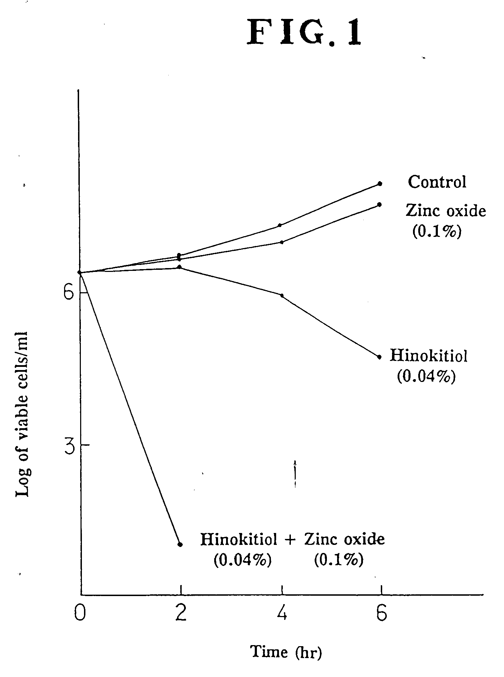

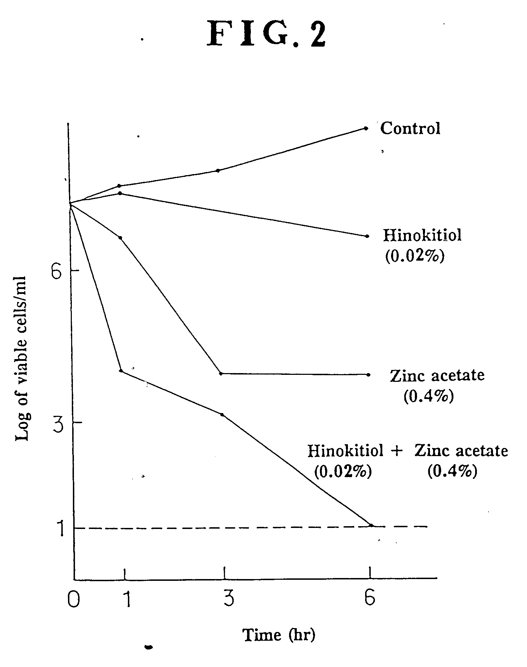

Viable cells were counted at timed intervals. The results obtained are shown in Fig. 1 and Fig. 2.

From Fig. 1 and Fig. 2, which are graphs showing the change in the number of viable cells counted with time, it is apparent that the use of hinokitiol in combination with zinc oxide or zinc acetate results in markedly increased antibacterial activity.

### Test Example 2 (Antibacterial activity test)

The effects of the combined use of hinokitiol and zinc acetate were examined by the checkerboard dilution method.

Hinokitiol SP and zinc acetate (Wako Pure Chemical Industries, Ltd.; Lot LKR 3395) were used as the test drugs. The following six bacterial strains were used as the test microorganisms.

### Bacterial strains used:

Staphylococcus (S.) aureus 209 PJC
S. aureus ATCC 6538 (Staphylococcus aureus)
S. epidermidis KPU1 (Staphylococcus epidermidis)
Escherichia coli K-12 (Escherichia coli)
Escherichia coli ATCC 8739 (Escherichia coli)
Bacillus subtilis IAM 1213 (Bacillus subtilis)

Each strain was precultured in Mueller-Hinton medium (incubation temperature: 37°C; incubation period: 18 to 20 hours), and the culture was diluted with Mueller-Hinton medium to 10⁸ cells/ml or 10⁶ cells/ml and the dilutions were used as the test bacterial cell suspensions.

The concentrations of the drug for testing and the method of preparing the drugs were as follows. Thus, an alcohol solution containing hinokitiol at a concentration of 5% was diluted with sterile distilled water to give a 0.4% hinokitiol solution. This solution was serially double-diluted with sterile distilled water to give solutions containing hinokitiol at concentrations of 2000 µg/ml, 1000 µg/ml, 500 µg/ml, 250 µg/ml, 125 µg/ml, 62.5 µg/ml, 31.3 µg/ml and 15.6 µg/ml. Separately, an aqueous solution containing zinc acetate at a concentration of 3.2% was serially double-diluted with sterile distilled water to give aqueous solution containing zinc acetate at concentrations of 16000 µg/ml, 8000 µg/ml, 4000 µg/ml, 2000 µg/ml, 1000 µg/ml, 500 µg/ml and 250 µg/ml.

When hinokitiol and zinc acetate were to be used combinedly, 0.5 ml each of the above two drug solutions containing respectively 20 times concentrations used were added, together with 9 ml of the medium, to each petri dish to obtain an agar plate. When hinokitiol and zinc acetate were to be used each individually, 1 ml of a 10-fold concentrated hinokitiol or zinc acetate solution and 9 ml of the medium were added to each petri dish to obtain an agar plate.

Mueller-Hinton plate media containing hinokitiol and/or zinc acetate at various concentrations were prepared by the above method and inoculated with each cell suspension with a cell concentration of 10⁸ cells/ml or 10⁶ cells/ml and, after incubating at 37°C for 48 hours, judgment was made as to whether cells had grown or not. The results obtained are shown in Table 1 through Table 12. In the following tables, "+" means that growth was observed, "-" means that no growth was observed, and "±" means that very slight growth was observed.

**Table 1**

| Bacterial strain used: Staphylococcus aureus 209PJC Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | ± |
| | 50 | - | - | - | - | - | - | - | + |
| | 25 | - | - | - | - | - | - | - | + |
| | 12.5 | - | - | - | - | - | - | + | + |
| | 6.25 | - | - | - | - | - | - | + | + |
| | 3.13 | - | - | - | - | - | + | + | + |
| | 1.56 | - | - | - | - | + | + | + | + |
| | 0 | - | - | + | + | + | + | + | |

**Table 2**

| Bacterial strain used: Staphylococcus aureus 209PJC Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | + |
| | 50 | - | - | - | - | - | - | - | + |
| | 25 | - | - | - | - | - | - | - | + |
| | 12.5 | - | - | - | - | - | - | - | + |
| | 6.25 | - | - | - | - | - | - | - | - |
| | 3.13 | - | - | - | - | - | - | - | - |
| | 1.56 | - | - | - | - | - | - | - | - |
| | 0 | - | - | + | + | + | + | + | |

**Table 3**

| Bacterial strain used: Staphylococcus aureus ATCC 6538 Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | + |
| | 50 | - | - | - | - | - | - | - | + |
| | 25 | - | - | - | - | - | - | - | + |
| | 12.5 | - | - | - | - | - | - | + | + |
| | 6.25 | - | - | - | - | - | - | - | - |
| | 3.13 | - | - | - | - | - | - | - | - |
| | 1.56 | - | - | - | - | + | - | - | - |
| | 0 | - | - | + | + | + | + | + | |

**Table 4**

| Bacterial strain used: Staphylococcus aureus ATCC 6538 Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | + |
| | 25 | - | - | - | - | - | - | - | + |
| | 12.5 | - | - | - | - | - | - | ± | + |
| | 6.25 | - | - | - | - | - | - | - | - |
| | 3.13 | - | - | - | - | - | - | - | - |
| | 1.56 | - | - | - | - | - | - | - | - |
| | 0 | - | - | + | + | + | + | + | |

**Table 5**

| Bacterial strain used: Staphylococcus epidermidis KPU 1 Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | + |
| | 50 | - | - | - | - | - | - | - | + |
| | 25 | - | - | - | - | - | - | - | + |
| | 12.5 | - | - | - | - | - | - | + | + |
| | 6.25 | - | - | - | - | - | - | - | - |
| | 3.13 | - | - | - | - | - | - | - | - |
| | 1.56 | - | - | - | - | - | - | - | - |
| | 0 | - | - | + | + | + | + | + | |

**Table 6**

| Bacterial strain used: Staphylococcus epidermidis KPU 1 Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | + |
| | 50 | - | - | - | - | - | - | - | + |
| | 25 | - | - | - | - | - | - | - | + |
| | 12.5 | - | - | - | - | - | - | - | + |
| | 6.25 | - | - | - | - | - | - | - | - |
| | 3.13 | - | - | - | - | - | - | - | - |
| | 1.56 | - | - | - | - | - | - | - | - |
| | 0 | - | - | + | + | + | + | + | |

**Table 7**

| Bacterial strain used: Escherichia coli K-12 Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | - |
| | 25 | - | - | - | - | - | - | - | - |
| | 12.5 | - | - | - | - | - | - | - | + |
| | 6.25 | - | - | - | - | + | + | + | + |
| | 3.13 | - | - | - | + | + | + | + | + |
| | 1.56 | - | - | + | + | + | + | + | + |
| | 0 | - | - | + | + | + | + | + | |

**Table 8**

| Bacterial strain used: Escherichia coli K-12 Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | - |
| | 25 | - | - | - | - | - | - | - | + |
| | 12.5 | - | - | - | - | - | - | - | + |
| | 6.25 | - | - | - | - | + | + | + | + |
| | 3.13 | - | - | - | + | + | + | + | + |
| | 1.56 | - | - | + | + | + | + | + | + |
| | 0 | - | - | + | + | + | + | + | |

**Table 9**

| Bacterial strain used: Escherichia coli ATCC 8739 Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | - |
| | 25 | - | - | - | - | - | - | - | + |
| | 12.5 | - | - | - | - | - | - | - | + |
| | 6.25 | - | - | - | - | + | + | + | + |
| | 3.13 | - | - | - | + | + | + | + | + |
| | 1.56 | - | - | - | + | + | + | + | + |
| | 0 | - | - | + | + | + | + | + | |

**Table 10**

| Bacterial strain used: Escherichia coli ATCC 8739 Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | - |
| | 25 | - | - | - | - | - | - | - | + |
| | 12.5 | - | - | - | - | - | - | - | + |
| | 6.25 | - | - | - | - | + | + | + | + |
| | 3.13 | - | - | - | - | + | + | + | + |
| | 1.56 | - | - | - | + | + | + | + | + |
| | 0 | - | - | - | + | + | + | + | |

**Table 11**

| Bacterial strain used: Bacillus subtilis IAM 1213 Inoculum size: 10⁸ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | + |
| | 25 | - | - | - | - | - | - | - | + |
| | 12.5 | - | - | - | - | - | - | - | + |
| | 6.25 | - | - | - | - | - | - | + | + |
| | 3.13 | - | - | - | - | - | - | - | + |
| | 1.56 | - | - | - | - | - | + | + | + |
| | 0 | - | - | + | + | + | + | + | |

**Table 12**

| Bacterial strain used: Bacillus subtilis IAM 1213 Inoculum size: 10⁶ cells/ml | | | | | | | | | |
|---|---|---|---|---|---|---|---|---|---|
| (Unit: µg/ml) | | | | | | | | | |

| | | Zinc acetate | | | | | | | |
|---|---|---|---|---|---|---|---|---|---|
| | | 1600 | 800 | 400 | 200 | 100 | 50 | 25 | 0 |
| Hinokitiol | 200 | - | - | - | - | - | - | - | - |
| | 100 | - | - | - | - | - | - | - | - |
| | 50 | - | - | - | - | - | - | - | + |
| | 25 | - | - | - | - | - | - | - | + |
| | 12.5 | - | - | - | - | - | - | - | + |
| | 6.25 | - | - | - | - | - | - | - | + |
| | 3.13 | - | - | - | - | - | - | - | + |
| | 1.56 | - | - | - | - | - | - | + | + |
| | 0 | - | - | - | + | + | + | + | |

From Tables 1 to 12 shown above, it is apparent that the combined use of hinokitiol and zinc acetate results in markedly improved antibacterial activity.

### Test Example 3 (Antibacterial activity test)

The effects of the combined use of hinokitiol and zinc chloride were examined after 24 hours of incubation at 37°C in the same manner as in Test Example 2 (except that the medium alone was changed, namely, Trypto Soy Bouillon medium was used as the preculture medium, dried bouillon medium for dilution, and heart infusion agar medium for plate preparation).

Hinokitiol SP and zinc chloride (Wako Pure Chemical Industries, Ltd.; Lot TWQ 1277) were used as the test drugs, and the following three bacterial strains as the test organisms.

### Strains used:

Staphylococcus aureus 209PJC
Bacillus subtilis PCI 219
Micrococcus luteus ATCC 9341

The results obtained are shown below in Table 13 through Table 15.

**Table 13**

| Bacterial strain used: Staphylococcus aureus 209PJC Inoculum size: 10⁶ cells/ml | | | | | | | | | | | | |
|---|---|---|---|---|---|---|---|---|---|---|---|---|
| (Unit: µg/ml) | | | | | | | | | | | | |

| | | Zinc chloride | | | | | | | | | | |
|---|---|---|---|---|---|---|---|---|---|---|---|---|
| | | 400 | 200 | 100 | 50 | 25 | 12.5 | 6.25 | 3.13 | 1.56 | 0.78 | 0 |
| Hinokitiol | 100 | - | - | - | - | - | - | - | - | - | - | - |
| | 50 | - | - | - | - | - | - | - | - | - | - | - |
| | 25 | - | - | - | - | - | - | - | + | + | + | + |
| | 12.5 | - | - | - | - | - | + | + | + | + | + | + |
| | 6.25 | - | - | - | - | - | - | - | - | - | - | - |
| | 3.13 | - | - | - | - | - | - | - | - | - | - | - |
| | 1.56 | - | - | - | - | - | - | - | - | - | - | - |
| | 0.78 | - | - | + | + | + | + | + | + | + | + | + |
| | 0.39 | - | - | + | + | + | + | + | + | + | + | + |
| | 0.20 | - | - | + | + | + | + | + | + | + | + | + |
| | 0 | - | - | + | + | + | + | + | + | + | + | |

**Table 14**

| Bacterial strain used: Bacillus subtilis PCI 219 Inoculum size: 10⁶ cells/ml | | | | | | | | | | | | |
|---|---|---|---|---|---|---|---|---|---|---|---|---|
| (Unit: µg/ml) | | | | | | | | | | | | |

| | | Zinc chloride | | | | | | | | | | |
|---|---|---|---|---|---|---|---|---|---|---|---|---|
| | | 400 | 200 | 100 | 50 | 25 | 12.5 | 6.25 | 3.13 | 1.56 | 0.78 | 0 |
| Hinokitiol | 100 | - | - | - | - | - | - | - | - | - | - | - |
| | 50 | - | - | - | - | - | - | - | - | - | - | - |
| | 25 | - | - | - | - | - | - | - | - | - | - | - |
| | 12.5 | - | - | - | - | - | - | + | + | + | + | + |
| | 6.25 | - | - | - | - | - | - | + | + | + | + | + |
| | 3.13 | - | - | - | - | - | - | + | + | + | + | + |
| | 1.56 | - | - | - | - | + | + | + | + | + | + | + |
| | 0.78 | - | + | + | + | + | + | + | + | + | + | + |
| | 0.39 | - | - | + | + | + | + | + | + | + | + | + |
| | 0.20 | - | - | + | + | + | + | + | + | + | + | + |
| | 0 | - | - | + | + | + | + | + | + | + | + | |

**Table 15**

| Bacterial strain used: Micrococcus luteus ATCC 9341 Inoculum size: 10⁶ cells/ml | | | | | | | | | | | | |
|---|---|---|---|---|---|---|---|---|---|---|---|---|
| (Unit: µg/ml) | | | | | | | | | | | | |

| | | Zinc chloride | | | | | | | | | | |
|---|---|---|---|---|---|---|---|---|---|---|---|---|
| | | 400 | 200 | 100 | 50 | 25 | 12.5 | 6.25 | 3.13 | 1.56 | 0.78 | 0 |
| Hinokitiol | 100 | - | - | - | - | - | - | - | - | - | - | - |
| | 50 | - | - | - | - | - | - | - | - | - | - | - |
| | 25 | - | - | - | - | - | - | - | - | - | - | + |
| | 12.5 | - | - | - | - | - | + | + | + | + | + | + |
| | 6.25 | - | - | - | - | + | + | + | + | + | + | + |
| | 3.13 | - | - | - | - | + | + | + | + | + | + | + |
| | 1.56 | - | - | + | + | + | + | + | + | + | + | + |
| | 0.78 | - | + | + | + | + | + | + | + | + | + | + |
| | 0.39 | - | + | + | + | + | + | + | + | + | + | + |
| | 0.20 | - | + | + | + | + | + | + | + | + | + | + |
| | 0 | - | + | + | + | + | + | + | + | + | + | |

### Test Example 4 (Antibacterial activity test)

The effects of the combined use of hinokitiol and zinc dipicolinate were investigated after 24 hours of incubation at 37°C in the same manner as in Test Example 2 (except that the medium alone was changed, namely Trypto Soy Bouillon medium was used as the preculture medium, dried bouillon medium for dilution, and heart infusion agar medium for plate preparation).

Hinokitiol SP and zinc dipicolinate were used as the test drugs and the following two bacterial strains as the test organisms.

### Strains used:

Staphylococcus epidermidis KPU-1
Bacillus subtilis ATCC 6633

The results obtained are shown below in Table 16 and Table 17.

**Table 16**

| Bacterial strain used: Staphylococcus epidermides KPU-1 Inoculum size: 10⁶ cells/ml | | | | | | | | | | | | |
|---|---|---|---|---|---|---|---|---|---|---|---|---|
| (Unit: µg/ml) | | | | | | | | | | | | |

| | | Zinc dipicolinate | | | | | | | | | | |
|---|---|---|---|---|---|---|---|---|---|---|---|---|
| | | 400 | 200 | 100 | 50 | 25 | 12.5 | 6.25 | 3.13 | 1.56 | 0.78 | 0 |
| Hinokitiol | 100 | - | - | - | - | - | - | - | - | - | - | - |
| | 50 | - | - | - | - | - | - | - | - | - | + | - |
| | 25 | - | - | - | - | - | + | + | + | + | + | + |
| | 12.5 | - | - | - | - | - | - | + | + | + | + | + |
| | 6.25 | - | - | - | - | - | - | - | - | - | - | - |
| | 3.13 | - | - | - | - | - | - | - | - | - | - | - |
| | 1.56 | - | - | - | - | - | - | - | - | - | - | - |
| | 0.78 | - | - | - | - | - | - | - | - | - | - | - |
| | 0.39 | - | - | - | - | - | - | - | - | - | - | - |
| | 0.20 | - | + | + | + | + | + | + | + | + | + | + |
| | 0 | + | + | + | + | + | + | + | + | + | + | |

**Table 17**

| Bacterial strain used: Bacillus subtilis ATCC 6633 Inoculum size: 10⁶ cells/ml | | | | | | | | | | | | |
|---|---|---|---|---|---|---|---|---|---|---|---|---|
| (Unit: µg/ml) | | | | | | | | | | | | |

| | | Zinc dipicolinate | | | | | | | | | | |
|---|---|---|---|---|---|---|---|---|---|---|---|---|
| | | 400 | 200 | 100 | 50 | 25 | 12.5 | 6.25 | 3.13 | 1.56 | 0.78 | 0 |
| Hinokitiol | 100 | - | - | - | - | - | - | - | - | - | - | - |
| | 50 | - | - | - | - | - | - | - | - | - | - | - |
| | 25 | - | - | - | - | - | - | - | - | + | + | + |
| | 12.5 | - | - | - | - | - | + | + | + | + | + | + |
| | 6.25 | - | - | - | - | + | + | + | + | + | + | + |
| | 3.13 | - | - | - | - | + | + | + | + | + | + | + |
| | 1.56 | - | - | + | + | + | + | + | + | + | + | + |
| | 0.78 | - | - | - | + | + | + | + | + | + | + | + |
| | 0.39 | - | + | + | + | + | + | + | + | + | + | + |
| | 0.20 | - | + | + | + | + | + | + | + | + | + | + |
| | 0 | - | + | + | + | + | + | + | + | + | + | |

## Claims

1. Use of a zinc compound and at least one member selected from the group consisting of hinokitiol and salts thereof for preparing a medicament effective in the treatment of an infectious disease caused by bacteria or fungi.

2. The use as claimed in claim 1, wherein said infectious disease is caused by bacteria.

3. The use as claimed in claim 1 or 2, wherein the ratio of said zinc compound (A) and said at least one member (B) selected from the group consisting of hinokitiol and salts thereof is A:B = 10 to 99.95:90 to 0.05 by weight.

4. The use as claimed in claim 3, wherein the ratio of said zinc compound (A) and said at least one member (B) selected from the group consisting of hinokitiol and salts thereof is A:B = 50 to 99.9:50 to 0.1 by weight.

5. The use as claimed in claim 1 or 2, wherein based on the total amount of said zinc compound and said at least one member selected from the group consisting of hinokitiol and salts thereof, said zinc compound is used in an amount of 10 to 99.95% by weight and said at least one member selected from the group consisting of hinokitiol and salts thereof is (are) used in an amount of 90 to 0.05% by weight.

6. The use as claimed in claim 5, wherein based on the total amount of said zinc compound and said at least one member selected from the group consisting of hinokitiol and salts thereof, said zinc compound is used in an amount of 50 to 99.9% by weight, and said at least one member selected from the group consisting of hinokitiol and salts thereof is (are) used in an amount of 50 to 0.1% by weight.

7. The use as claimed in any one of claims 1 to 6, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paraphenolsulfonate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc phenolsulfonate, zinc sebacate, sodium zinc tripolyphospate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc dipicolinate, zinc-glycerol complex, zinc-bishistidine complex, zinc-3,4-dihydroxybenzoic acid complex, zinc nicotinate and zinc-nicotinamide complex.

8. The use as claimed in claim 7, wherein said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

9. The use as claimed in claim 8, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc-glycerol complex, zinc-bishistidine complex, zinc-3,4-dihydroxybenzoic acid complex, zinc nicotinate and zinc-nicotinamide complex and said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

10. The use as claimed in claim 1 or 2, wherein based on the total amount of said zinc compound (a) and said at least one member (b) selected from the group consisting of hinokitiol and salts thereof, said zinc compound (a) is used in an amount of 50 to 99.9% by weight and said at least one member (b) selected from the group consisting of hinokitiol and salts thereof is (are) used in an amount of 50 to 0.1% by weight, said zinc compound (a) being at least one member selected from the group consisting of zinc oxide, zinc acetate, zinc pyrithione, hinokitiolato zinc, zinc chloride and zinc dipicolinate and said component (b) being at least one member selected from the group consisting of hinokitiol and hinokitiol sodium salt.

11. The use as claimed in claim 10, wherein based on the total amount of said zinc compound (a) and said at least one member (b) selected from the group consisting of hinokitiol and salts thereof, said zinc compound (a) is used in an amount of 50 to 99.9% by weight and said at least one member (b) selected from the group consisting of hinokitiol and salts thereof is (are) used in an amount of 50 to 0.1% by weight, said zinc compound (a) being at least one member selected from the group consisting of zinc oxide, zinc acetate, zinc pyrithione, hinokitiolato zinc and zinc chloride and said component (b) being at least one member selected from the group consisting of hinokitiol and hinokitiol sodium salt.

12. The use as claimed in Claim 1 or 2, wherein said zinc compound is zinc oxide, and based on the total amount of zinc oxide and said at least one member selected from the group consisting of hinokitiol and salts thereof, zinc oxide is used in an amount of 10 to 99.95% by weight and said at least one member selected from the group consisting of hinokitiol and salts thereof is (are) used in an amount of 90 to 0.05% by weight.

13. The use as claimed in Claim 12, wherein said zinc compound is zinc oxide, and based on the total amount of zinc oxide and said at least one member selected from the group consisting of hinokitiol and salts thereof, zinc oxide is used in an amount of 50 to 99.9% by weight and said at least one member selected from the group consisting of hinokitiol and salts thereof is (are) used in an amount of 50 to 0.1% by weight.

14. The use as claimed in Claim 1 or 2, wherein said zinc compound is zinc pyrithione, and based on the total amount of zinc pyrithione and said at least one member selected from the group consisting of hinokitiol and salts thereof, zinc pyrithione is used in an amount of 10 to 99.95% by weight and said at least one member selected from the group consisting of hinokitiol and salts thereof is (are) used in an amount of 90 to 0.05% by weight.

15. The use as claimed in Claim 14, wherein said zinc compound is zinc pyrithione, and based on the total amount of zinc pyrithione and said at least one member selected from the group consisting of hinokitiol and salts thereof, zinc pyrithione is used in an amount of 50 to 99.9% by weight and said at least one member selected from the group consisting of hinokitiol and salts thereof is (are) used in an amount of 50 to 0.1% by weight.

16. The use as claimed in Claim 1 or 2, wherein said zinc compound is zinc myristate, and based on the total amount of zinc myristate and said at least one member selected from the group consisting of hinokitiol and salts thereof, zinc myristate is used in an amount of 10 to 99.95% by weight and said at least one member selected from the group consisting of hinokitiol and salts thereof is (are) used in an amount of 90 to 0.05% by weight.

17. The use as claimed in Claim 16, wherein said zinc compound is zinc myristate, and based on the total amount of zinc myristate and said at least one member selected from the group consisting of hinokitiol and salts thereof, zinc myristate is used in an amount of 50 to 99.9% by weight and said at least one member selected from the group consisting of hinokitiol and salts thereof is(are) used in an amount of 50 to 0.1% by weight.

18. The use as claimed in claim 13, wherein based on the total amount of zinc oxide and hinokitiol, zinc oxide is used in an amount of 50 to 99.9% by weight and hinokitiol is used in an amount of 50 to 0.1% by weight.

19. A method of preserving a cosmetic which comprises causing said cosmetic to contain a zinc compound and at least one member selected from the group consisting of hinokitiol and salts thereof.

20. A method as claimed in Claim 19, wherein the ratio of said zinc compound (A) and said at least one member (B) selected from the group consisting of hinokitiol and salts thereof is A:B = 10 to 99.95:90 to 0.05 by weight.

21. A method as claimed in Claim 20, wherein the ratio of said zinc compound (A) and said at least one member (B) selected from the group consisting of hinokitiol and salts thereof is A:B = 50 to 99.9:50 to 0.1 by weight.

22. A method as claimed in Claim 19, wherein based on the total amount of said zinc compound and said at least one member selected from the group consisting of hinokitiol and salts thereof, said zinc compound is used in an amount of 10 to 99.95% by weight and said at least one member selected from the group consisting of hinokitiol and salts thereof is (are) used in an amount of 90 to 0.05% by weight.

23. A method as claimed in Claim 22, wherein based on the total amount of said zinc compound and said at least one member selected from the group consisting of hinokitiol and salts thereof, said zinc compound is used in an amount of 50 to 99.9% by weight, and said at least one member selected from the group consisting of hinokitiol and salts thereof is (are) used in an amount of 50 to 0.1% by weight.

24. A method as claimed in any one of Claims 19 to 23, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paraphenolsulfonate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc phenolsulfonate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc dipicolinate, zinc-glycerol complex, zinc-bishistidine complex, zinc-3,4-dihydroxybenzoic acid complex, zinc nicotinate and zinc-nicotinamide complex.

25. A method as claimed in Claim 24, wherein said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

26. A method as claimed in claim 25, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc-glycerol complex, zinc-bishistidine complex, zinc-3,4-dihydroxybenzoic acid complex, zinc nicotinate and zinc-nicotinamide complex and said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

27. A method as claimed in Claim 19, wherein based on the total amount of said zinc compound (a) and said at least one member selected from the group consisting of hinokitiol and salts thereof, said zinc compound (a) is used in an amount of 50 to 99.9% by weight and said at least one member selected from the group consisting of hinokitiol and salts thereof is(are) used in an amount of 50 to 0.1% by weight, said zinc compound (a) being at least one member selected from the group consisting of zinc oxide, zinc acetate, zinc pyrithione, zinc hinokitiolato zinc, zinc chloride and zinc dipicolinate and said component (b) being at least one member selected from the group consisting of hinokitiol and hinokitiol sodium salt.

28. A method as claimed in Claim 27, wherein based on the total amount of said zinc compound (a) and said at least one member selected from the group consisting of hinokitiol and salts thereof, said zinc compound (a) is used in an amount of 50 to 99.9% by weight and said at least one member selected from the group consisting of hinokitiol and salts thereof is(are) used in an amount of 50 to 0.1% by weight, said zinc compound (a) being at least one member selected from the group consisting of zinc oxide, zinc acetate, zinc pyrithione, zinc hinokitiolato zinc and zinc chloride and said component (b) being at least one member selected from the group consisting of hinokitiol and hinokitiol sodium salt.

29. A method as claimed in Claim 19, wherein said zinc compound is zinc oxide, and based on the total amount of zinc oxide and said at least one member selected from the group consisting of hinokitiol and salts thereof, zinc oxide is used in an amount of 10 to 99.95% by weight and said at least one member selected from the group consisting of hinokitiol and salts thereof is(are) used in an amount of 90 to 0.05% by weight.

30. A method as claimed in Claim 29, wherein said zinc compound is zinc oxide, and based on the total amount of zinc oxide and said at least one member selected from the group consisting of hinokitiol and salts thereof, zinc oxide is used in an amount of 50 to 99.9% by weight and said at least one member selected from the group consisting of hinokitiol and salts thereof is(are) used in an amount of 50 to 0.1% by weight.

31. A method as claimed in Claim 19, wherein said zinc compound is zinc pyrithione, and based on the total amount of zinc pyrithione and said at least one member selected from the group consisting of hinokitiol and salts thereof, zinc pyrithione is used in an amount of 10 to 99.95% by weight and said at least one member selected from the group consisting of hinokitiol and salts thereof is(are) used in an amount of 90 to 0.05% by weight.

32. A method as claimed in Claim 31, wherein said zinc compound is zinc pyrithione, and based on the total amount of zinc pyrithione and said at least one member selected from the group consisting of hinokitiol and salts thereof, zinc pyrithione is used in an amount of 50 to 99.9% by weight and said at least one member selected from the group consisting of hinokitiol and salts thereof is(are) used in an amount of 50 to 0.1% by weight.

33. A method as claimed in Claim 19, wherein said zinc compound is zinc myristate, and based on the total amount of zinc myristate and said at least one member selected from the group consisting of hinokitiol and salts thereof, zinc myristate is used in an amount of 10 to 99.95% by weight and said at least one member selected from the group consisting of hinokitiol and salts thereof is(are) used in an amount of 90 to 0.05% by weight.

34. A method as claimed in Claim 33, wherein said zinc compound is zinc myristate, and based on the total amount of zinc myristate and said at least one member selected from the group consisting of hinokitiol and salts thereof, zinc myristate is used in an amount of 50 to 99.9% by weight and said at least one member selected from the group consisting of hinokitiol and salts thereof is(are) used in an amount of 50 to 0.1% by weight.

35. A method as claimed in claim 30, wherein based on the total amount of zinc oxide and hinokitiol, zinc oxide is used in an amount of 50 to 99.9% by weight and hinokitiol is used in an amount of 50 to 0.1% by weight.

36. An antibacterial and or antifungal agent which comprises a zinc compound and at least one member selected from the group consisting of hinokitiol and salts thereof with the proviso that the zinc compound is neither zinc dipicolinate, a zinc salt of edetic acid, zinc phenolsulfonate nor zinc paraphenolsulfonate.

37. An antibacterial and/or antifungal agent as claimed in Claims 36, wherein based on the total amount of said zinc compound and said at least one member selected from the group consisting of hinokitiol and salts thereof, said zinc compound is used in an amount of 10 to 99.95% by weight and said at least one member selected from the group consisting of hinokitiol and salts thereof is(are) used in an amount of 90 to 0.05% by weight.

38. An antibacterial and/or antifungal agent as claimed in Claim 37, wherein based on the total amount of said zinc compound and said at least one member selected from the group consisting of hinokitiol and salts thereof, said zinc compound is used in an amount of 50 to 99.9% by weight, and said at least one member selected from the group consisting of hinokitiol and salts thereof is(are) used in an amount of 50 to 0.1% by weight.

39. An antibacterial and/or antifungal agent as claimed in Claims 36, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc-glycerol complex, zinc-bishistidine complex, zinc-3,4-dihydroxybenzoic acid complex, zinc nicotinate and zinc-nicotinamide complex and said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

40. An antibacterial and/or antifungal agent as claimed in Claim 37, wherein said agent comprises zinc oxide and said at least one member selected from the group consisting of hinokitiol and salts thereof, said agent contains zinc oxide and said at least one member selected from the group consisting of hinokitiol or salts thereof in a total amount of 0.001 to 20% by weight, and based on the total amount of zinc oxide and said at least one member selected from the group consisting of hinokitiol and salts thereof, zinc oxide is used in an amount of 10 to 99.95% by weight and said at least one member selected from the group consisting of hinokitiol and salts thereof is(are) used in an amount of 90 to 0.05% by weight.

41. An antibacterial and/or antifungal agent as claimed in Claim 40, wherein said agent comprises zinc oxide and said at least one member selected from the group consisting of hinokitiol and salts thereof, said agent contains zinc oxide and said at least one member selected from the group consisting of hinokitiol or salts thereof in a total amount of 0.01 to 15% by weight, and based on the total amount of zinc oxide and said at least one member selected from the group consisting of hinokitiol and salts thereof, zinc oxide is used in an amount of 50 to 99.9% by weight and said at least one member selected from the group consisting of hinokitiol and salts thereof is(are) used in an amount of 50 to 0.1% by weight.

42. An antibacterial and/or antifungal agent as claimed in Claim 36, wherein said agent comprises zinc pyrithione and said at least one member selected from the group consisting of hinokitiol and salts thereof, said agent contains said zinc pyrithione and said at least one member selected from the group consisting of hinokitiol or salts thereof in a total amount of 0.001 to 20% by weight, and based on the total amount of said zinc pyrithione and said at least one member selected from the group consisting of hinokitiol and salts thereof, said zinc pyrithione is used in an amount of 10 to 99.95% by weight and said at least one member selected from the group consisting of hinokitiol and salts thereof is(are) used in an amount of 90 to 0.05% by weight.

43. An antibacterial and/or antifungal agent as claimed in Claim 42, wherein said agent comprises zinc pyrithione and said at least one member selected from the group consisting of hinokitiol and salts thereof, said agent contains said zinc pyrithione and said at least one member selected from the group consisting of hinokitiol or salts thereof in a total amount of 0.01 to 15% by weight, and based on the total amount of said zinc pyrithione and said at least one member selected from the group consisting of hinokitiol and salts thereof, said zinc pyrithione is used in an amount of 50 to 99.9% by weight and said at least one member selected from the group consisting of hinokitiol and salts thereof is(are) used in an amount of 50 to 0.1% by weight.

44. An antibacterial and/or antifungal agent as claimed in Claim 36, wherein said agent comprises zinc myristate and said at least one member selected from the group consisting of hinokitiol and salts thereof, said agent contains said zinc myristate and said at least one member selected from the group consisting of hinokitiol or salts thereof in a total amount of 0.001 to 20% by weight, and based on the total amount of said zinc myristate and said at least one member selected from the group consisting of hinokitiol and salts thereof, said zinc myristate is used in an amount of 10 to 99.95% by weight and said at least one member selected from the group consisting of hinokitiol and salts thereof is(are) used in an amount of 90 to 0.05% by weight.

45. An antibacterial and/or antifungal agent as claimed in Claim 44, wherein said agent comprises zinc myristate and said at least one member selected from the group consisting of hinokitiol and salts thereof, said agent contains said zinc myristate and said at least one member selected from the group consisting of hinokitiol or salts thereof in a total amount of 0.01 to 15% by weight, and based on the total amount of said zinc myristate and said at least one member selected from the group consisting of hinokitiol and salts thereof, said zinc myristate is used in an amount of 50 to 99.9% by weight and said at least one member selected from the group consisting of hinokitiol and salts thereof is(are) used in an amount of 50 to 0.1% by weight.

46. A cosmetic which contains a zinc compound and at least one member selected from the group consisting of hinokitiol and salts thereof under the proviso that the zinc compound is not represented by zinc paraphenolsulfonate, zinc phenolsulfonate, zinc dipicolinate or a zinc salt of edetic acid.

47. A cosmetic as claimed in Claim 46, wherein said zinc compound and said at least one member selected from the group consisting of hinokitiol and salts thereof are incorporated therein as an antidandruff agent, bactericide and/or preservative.

48. A cosmetic as claimed in Claim 47, wherein the ratio of said zinc compound (A) and said at least one member (B) selected from the group consisting of hinokitiol and salts thereof is A:B = 10 to 99.95:90 to 0.05 by weight.

49. A cosmetic as claimed in Claim 48, wherein the ratio of said zinc compound (A) and said at least one member (B) selected from the group consisting of hinokitiol and salts thereof is A:B = 50 to 99.9:50 to 0.1 by weight.

50. A cosmetic as claimed in Claim 47, wherein based on the total amount of said zinc compound and said at least one member selected from the group consisting of hinokitiol and salts thereof, said zinc compound is used in an amount of 10 to 99.95% by weight and said at least one member selected from the group consisting of hinokitiol and salts thereof is(are) used in an amount of 90 to 0.05% by weight.

51. A cosmetic as claimed in Claim 50, wherein based on the total amount of said zinc compound and said at least one member selected from the group consisting of hinokitiol and salts thereof, said zinc compound is used in an amount of 50 to 99.9% by weight, and said at least one member selected from the group consisting of hinokitiol and salts thereof is(are) used in an amount of 50 to 0.1% by weight.

52. A cosmetic as claimed in any one of Claims 47 to 51, wherein said zinc compound is at least one member selected from the group consisting of zinc oxide, zinc chloride, zinc nitrate, zinc sulfate, zinc phosphate, zinc aluminate, zinc fluoride, zinc iodide, zinc hydroxide, zinc carbonate, zinc chromate, zinc benzoate, zinc acetate, zinc paraaminobenzoate, zinc paradimethylaminobenzoate, zinc paramethoxycinnamate, zinc lactate, zinc-2-mercaptopyridine N-oxide complex, zinc gluconate, zinc picrate, zinc citrate, zinc aspartate, zinc naphthenate, zinc salicylate, zinc sebacate, sodium zinc tripolyphosphate, zinc stearate, zinc caprate, zinc laurate, zinc myristate, zinc palmitate, zinc oleate, zinc polyphosphonate, zinc chondroitinsulfate, zinc undecylenate, zinc ascorbate, zinc pyrithione, hinokitiolato zinc, zinc-glycerol complex, zinc-bishistidine complex, zinc-3,4-dihydroxybenzoic acid complex, zinc nicotinate and zinc-nicotinamide complex.

53. A cosmetic as claimed in Claim 52, herein said at least one member selected from the group consisting of hinokitiol and salts thereof is at least one member selected from the group consisting of hinokitiol and sodium salt, potassium salt, magnesium salt, copper salt, zinc salt, diethanolamine salt, 2-amino-2-ethyl-1,3-propanediol salt, triethanolamine salt, morpholine salt, piperazine salt, piperidine salt, ammonium salt, arginine salt, lysine salt and histidine salt of hinokitiol.

54. A cosmetic as claimed in Claim 47, wherein based on the total amount of said zinc compound (a) and said at least one member (b) selected from the group consisting of hinokitiol and salts thereof, said zinc compound (a) is used in an amount of 50 to 99.9% by weight and said at least one member (b) selected from the group consisting of hinokitiol and salts thereof is(are) used in an amount of 50 to 0.1% by weight, said zinc compound (a) being at least one member selected from the group consisting of zinc oxide, zinc acetate, zinc pyrithione, hinokitiolato zinc and zinc chloride and said component (b) being at least one member selected from the group consisting of hinokitiol and hinokitiol sodium salt.

55. A cosmetic as claimed in Claim 46, wherein said cosmetic comprises zinc oxide and said at least one member selected from the group consisting of hinokitiol and salts thereof, said cosmetic contains zinc oxide and said at least one member selected from the group consisting of hinokitiol or salts thereof in a total amount of 0.0001 to 99.9% by weight, and based on the total amount of zinc oxide and said at least one member selected from the group consisting of hinokitiol and salts thereof, zinc oxide is used in an amount of 10 to 99.95% by weight and said at least one member selected from the group consisting of hinokitiol and salts thereof is(are) used in an amount of 90 to 0.05% by weight.

56. A cosmetic as claimed in Claim 55, wherein said cosmetic comprises zinc oxide and said at least one member selected from the group consisting of hinokitiol and salts thereof, said cosmetic contains zinc oxide and said at least one member selected from the group consisting of hinokitiol or salts thereof in a total amount of 0.001 to 30% by weight, and based on the total amount of zinc oxide and said at least one member selected from the group consisting of hinokitiol and salts thereof, zinc oxide is used in an amount of 50 to 99.9% by weight and said at least one member selected from the group consisting of hinokitiol and salts thereof is(are) used in an amount of 50 to 0.1% by weight.

57. A cosmetic as claimed in Claim 46, wherein said cosmetic comprises zinc pyrithione and said at least one member selected from the group consisting of hinokitiol and salts thereof, said cosmetic contains said zinc pyrithione and said at least one member selected from the group consisting of hinokitiol or salts thereof in a total amount of 0.0001 to 99.9% by weight, and based on the total amount of said zinc pyrithione and said at least one member selected from the group consisting of hinokitiol and salts thereof, said zinc pyrithione is used in an amount of 10 to 99.95% by weight and said at least one member selected from the group consisting of hinokitiol and salts thereof is(are) used in an amount of 90 to 0.05% by weight.

58. A cosmetic as claimed in Claim 57, wherein said cosmetic comprises zinc pyrithione and said at least one member selected from the group consisting of hinokitiol and salts thereof, said cosmetic contains said zinc pyrithione and said at least one member selected from the group consisting of hinokitiol or salts thereof in a total amount of 0.001 to 30% by weight, and based on the total amount of said zinc pyrithione and said at least one member selected from the group consisting of hinokitiol and salts thereof, said zinc pyrithione is used in an amount of 50 to 99.9% by weight and said at least one member selected from the group consisting of hinokitiol and salts thereof is(are) used in an amount of 50 to 0.1% by weight.

59. A cosmetic as claimed in Claim 46, wherein said cosmetic comprises zinc myristate and said at least one member selected from the group consisting of hinokitiol and salts thereof, said cosmetic contains said zinc myristate and said at least one member selected from the group consisting of hinokitiol or salts thereof in a total amount of 0.0001 to 99.9% by weight, and based on the total amount of said zinc myristate and said at least one member selected from the group consisting of hinokitiol and salts thereof, said zinc myristate is used in an amount of 10 to 99.95% by weight and said at least one member selected from the group consisting of hinokitiol and salts thereof is(are) used in an amount of about 90 to 0.05% by weight.

60. A cosmetic as claimed in Claim 59, wherein said cosmetic comprises zinc myristate and said at least one member selected from the group consisting of hinokitiol and salts thereof, said cosmetic contains said zinc myristate and said at least one member selected from the group consisting of hinokitiol or salts thereof in a total amount of 0.001 to 30% by weight, and based on the total amount of said zinc myristate and said at least one member selected from the group consisting of hinokitiol and salts thereof, said zinc myristate is used in an amount of 50 to 99.9% by weight and said at least one member selected from the group consisting of hinokitiol and salts thereof is(are) used in an amount of 50 to 0.1% by weight.

61. A cosmetic as claimed in Claim 54, wherein based on the total amount of zinc oxide and hinokitiol, zinc oxide is used in an amount of 50 to 99.9% by weight and hinokitiol in an amount of 50 to 0.1% by weight.

62. Method of preparing a medicament effective in the treatment of an infectious disease caused by bacteria or fungi comprising the step of incorporating a zinc compound and at least one member selected from the group consisting of hinokitiol and salts thereof into a pharmaceutically acceptable diluent or exipient provided that the zinc compound is not a zinc salt of edetic acid.

## Patentansprüche

1. Verwendung einer Zinkverbindung und mindestens eines Mitglieds, ausgewählt aus der Gruppe, bestehend aus Hinokitiol und seinen Salzen, zur Herstellung eines Medikaments, das bei der Behandlung einer durch Bakterien oder Pilze verursachten Infektionskrankheit wirksam ist.

2. Verwendung gemäß Anspruch 1, worin die Infektionskrankheit durch Bakterien verursacht wird.

3. Verwendung gemäß Anspruch 1 oder 2, worin das Verhältnis der Zinkverbindung (A) und des mindestens einen Mitglieds (B), ausgewählt aus der Gruppe, bestehend aus Hinokitiol und seinen Salzen, A:B = 10 bis 99,95:90 bis 0,05 per Gewicht beträgt.

4. Verwendung gemäß Anspruch 3, worin das Verhältnis der Zinkverbindung (A) und des mindestens einen Mitglieds (B), ausgewählt aus der Gruppe, bestehend aus Hinokitiol und seinen Salzen, A:B = 50 bis 99,9:50 bis 0,1 per Gewicht beträgt.

5. Verwendung gemäß Anspruch 1 oder 2, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

6. Verwendung gemäß Anspruch 5, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

7. Verwendung gemäß einem der Ansprüche 1 bis 6, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparaphenolsulfonat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinkphenolsulfonat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zinkdipicolinat, Zink-Glycerin-Komplex, Zink-Bishistidin-Komplex, Zink-3,4-dihydroxybenzoesäure-Komplex, Zinknicotinat und Zink-Nicotinamid-Komplex ist.

8. Verwendung gemäß Anspruch 7, worin das mindestens eine Mitglied, ausgewählt aus der Gruppe, bestehend aus Hinokitiol und seinen Salzen, mindestens ein Mitglied, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols ist.

9. Verwendung gemäß Anspruch 8, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zink-Glycerin-Komplex, Zink-Bishistidin-Komplex, Zink-3,4-dihydroxybenzoesäure-Komplex, Zinknicotinat und Zink-Nicotinamid-Komplex und das mindestens eine Mitglied, ausgewählt aus der Gruppe, bestehend aus Hinokitiol und seinen Salzen, mindestens ein Mitglied ist, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols.

10. Verwendung gemäß Anspruch 1 oder 2, worin, bezogen auf die Gesamtmenge der Zinkverbindung (a) und dem mindestens einen Mitglied (b), ausgewählt aus der Gruppe, bestehend aus Hinokitiol und seinen Salzen, die Zinkverbindung (a) in einer Menge von 50 bis 99,9 Gew.-% und das mindestens eine Mitglied (b), ausgewählt aus der Gruppe, bestehend aus Hinokitiol und seinen Salzen, in einer Menge von 50 bis 0,1 Gew.-% verwendet wird (werden), wobei die Zinkverbindung (a) mindestens ein Mitglied, ausgewählt aus der Gruppe, bestehend aus Zinkoxid, Zinkacetat, Zinkpyrithion, Hinokitiolatozink, Zinkchlorid und Zinkdipicolinat und die Verbindung (b) mindestens ein Mitglied, ausgewählt aus der Gruppe, bestehend aus Hinokitiol und Hinokitiol-Natriumsalz ist.

11. Verwendung gemäß Anspruch 10, worin, bezogen auf die Gesamtmenge der Zinkverbindung (a) und dem mindestens einen Mitglied (b), ausgewählt aus der Gruppe, bestehend aus Hinokitiol und seinen Salzen, die Zinkverbindung (a) in einer Menge von 50 bis 99,9 Gew.-% und das mindestens eine Mitglied (b), ausgewählt aus der Gruppe, bestehend aus Hinokitiol und seinen Salzen, in einer Menge von 50 bis 0,1 Gew.-% verwendet wird (werden), wobei die Zinkverbindung (a) mindestens ein Mitglied, ausgewählt aus der Gruppe, bestehend aus Zinkoxid, Zinkacetat, Zinkpyrithion, Hinokitiolatozink und Zinkchlorid und die Verbindung (b) mindestens ein Mitglied, ausgewählt aus der Gruppe, bestehend aus Hinokitiol und dem Hinokitiol-Natriumsalz ist.

12. Verwendung gemäß Anspruch 1 oder 2, worin die Zinkverbindung Zinkoxid ist, und, bezogen auf die Gesamtmenge des Zinkoxids und des mindestens einen Mitglieds, ausgewählt aus der Gruppe, bestehend aus Hinokitiol und seinen Salzen, Zinkoxid in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

13. Verwendung gemäß Anspruch 12, worin die Zinkverbindung Zinkoxid ist, und, bezogen auf die Gesamtmenge des Zinkoxids und des mindestens einen Mitglieds, ausgewählt aus der Gruppe, bestehend aus Hinokitiol und seinen Salzen, Zinkoxid in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

14. Verwendung gemäß Anspruch 1 oder 2, worin die Zinkverbindung Zinkpyrithion ist, und, bezogen auf die Gesamtmenge des Zinkpyrithions und des mindestens einen Mitglieds, ausgewählt aus der Gruppe, bestehend aus Hinokitiol und seinen Salzen, Zinkpyrithion in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

15. Verwendung gemäß Anspruch 14, worin die Zinkverbindung Zinkpyrithion ist, und, bezogen auf die Gesamtmenge des Zinkpyrithions und des mindestens einen Mitglieds, ausgewählt aus der Gruppe, bestehend aus Hinokitiol und seinen Salzen, Zinkpyrithion in einer Menge von 50 bis 99,9 Gew.-% und das mindestens eine Mitglied ausgewählt aus der Gruppe, bestehend aus Hinokitiol und seinen Salzen, in einer Menge von 50 bis 0,1 Gew.-% verwendet wird (werden).

16. Verwendung gemäß Anspruch 1 oder 2, worin die Zinkverbindung Zinkmyristat ist, und, bezogen auf die Gesamtmenge des Zinkmyristats und des mindestens einen Mitglieds, ausgewählt aus der Gruppe, bestehend aus Hinokitiol und seinen Salzen, Zinkmyristat in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

17. Verwendung gemäß Anspruch 16, worin die Zinkverbindung Zinkmyristat ist, und bezogen auf die Gesamtmenge des Zinkmyristats und des mindestens einen Mitglieds ausgewählt aus der Gruppe, bestehend aus Hinokitiol und seinen Salzen, Zinkmyristat in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

18. Verwendung gemäß Anspruch 13, worin, bezogen auf die Gesamtmenge des Zinkoxids und Hinokitiols, Zinkoxid in einer Menge von 50 bis 99,9 Gew.-% und Hinokitiol in einer Menge von 50 bis 0,1 Gew.-% verwendet wird.

19. Verfahren zum Haltbarmachen eines Kosmetikums, welches umfaßt, daß man das Kosmetikum eine Zinkverbindung und mindestens ein Mitglied, ausgewählt aus der Gruppe, bestehend aus Hinokitiol und seinen Salzen, enthalten läßt.

20. Verfahren gemäß Anspruch 19, worin das Verhältnis der Zinkverbindung (A) und des mindestens einen Mitglieds (B), ausgewählt aus der Gruppe, bestehend aus Hinokitiol und seinen Salzen, A:B = 10 bis 99,95:90 bis 0,05 per Gewicht beträgt.

21. Verfahren gemäß Anspruch 20, worin das Verhältnis der Zinkverbindung (A) und des mindestens einen Mitglieds (B), ausgewählt aus der Gruppe, bestehend aus Hinokitiol und seinen Salzen, A:B = 50 bis 99,9:50 bis 0,1 per Gewicht beträgt.

22. Verfahren gemäß Anspruch 19, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

23. Verfahren gemäß Anspruch 22, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

24. Verfahren gemäß einem der Ansprüche 19 bis 23, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparaphenolsulfonat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinkphenolsulfonat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zinkdipicolinat, Zink-Glycerin-Komplex, Zink-Bishistidin-Komplex, Zink-3,4-dihydroxybenzoesäure-Komplex, Zinknicotinat und Zink-Nicotinamid-Komplex ist.

25. Verfahren gemäß Anspruch 24, worin das mindestens eine Mitglied, ausgewählt aus der Gruppe, bestehend aus Hinokitiol und seinen Salzen, mindestens ein Mitglied, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols ist.

26. Verwendung gemäß Anspruch 25, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zink-Glycerin-Komplex, Zink-Bishistidin-Komplex, Zink-3,4-dihydroxybenzoesäure-Komplex, Zinknicotinat und Zink-Nicotinamid-Komplex und das mindestens eine Mitglied, ausgewählt aus der Gruppe, bestehend aus Hinokitiol und seinen Salzen, mindestens ein Mitglied ist, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols.

27. Verfahren gemäß Anspruch 19, worin, bezogen auf die Gesamtmenge der Zinkverbindung (a) und dem mindestens einen Mitglied, ausgewählt aus der Gruppe, bestehend aus Hinokitiol und seinen Salzen, die Zinkverbindung (a) in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden), wobei die Zinkverbindung (a) mindestens ein Mitglied, ausgewählt aus der Gruppe, bestehend aus Zinkoxid, Zinkacetat, Zinkpyrithion, Hinokitiolatozink, Zinkchlorid und Zinkdipicolinat und die Verbindung (b) mindestens ein Mitglied, ausgewählt aus der Gruppe, bestehend aus Hinokitiol und Hinokitiol-Natriumsalz ist.

28. Verfahren gemäß Anspruch 27, worin, bezogen auf die Gesamtmenge der Zinkverbindung (a) und dem mindestens einen Mitglied, ausgewählt aus der Gruppe, bestehend aus Hinokitiol und seinen Salzen, die Zinkverbindung (a) in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden), wobei die Zinkverbindung (a) mindestens ein Mitglied, ausgewählt aus der Gruppe, bestehend aus Zinkoxid, Zinkacetat, Zinkpyrithion, Hinokitiolatozink und Zinkchlorid und die Verbindung (b) mindestens ein Mitglied, ausgewählt aus der Gruppe, bestehend aus Hinokitiol und Hinokitiol-Natriumsalz ist.

29. Verfahren gemäß Anspruch 19, worin die Zinkverbindung Zinkoxid ist, und, bezogen auf die Gesamtmenge des Zinkoxids und des mindestens einen Mitglieds, ausgewählt aus der Gruppe, bestehend aus Hinokitiol und seinen Salzen, Zinkoxid in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

30. Verfahren gemäß Anspruch 29, worin die Zinkverbindung Zinkoxid ist, und, bezogen auf die Gesamtmenge des Zinkoxids und des mindestens einen Mitglieds, ausgewählt aus der Gruppe, bestehend aus Hinokitiol und seinen Salzen, Zinkoxid in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

31. Verfahren gemäß Anspruch 19, worin die Zinkverbindung Zinkpyrithion ist, und, bezogen auf die Gesamtmenge des Zinkpyrithions und des mindestens einen Mitglieds, ausgewählt aus der Gruppe, bestehend aus Hinokitiol und seinen Salzen, Zinkpyrithion in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

32. Verfahren gemäß Anspruch 31, worin die Zinkverbindung Zinkpyrithion ist, und, bezogen auf die Gesamtmenge des Zinkpyrithions und des mindestens einen Mitglieds, ausgewählt aus der Gruppe, bestehend aus Hinokitiol und seinen Salzen, Zinkpyrithion in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

33. Verfahren gemäß Anspruch 19, worin die Zinkverbindung Zinkmyristat ist, und bezogen auf die Gesamtmenge des Zinkmyristats und des mindestens einen Mitglieds, ausgewählt aus der Gruppe, bestehend aus Hinokitiol und seinen Salzen, Zinkmyristat in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

34. Verfahren gemäß Anspruch 33, worin die Zinkverbindung Zinkmyristat ist, und, bezogen auf die Gesamtmenge des Zinkmyristats und des mindestens einen Mitglieds, ausgewählt aus der Gruppe, bestehend aus Hinokitiol und seinen Salzen, Zinkmyristat in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

35. Verfahren gemäß Anspruch 30, worin, bezogen auf die Gesamtmenge des Zinkoxids und Hinokitiols, Zinkoxid in einer Menge von 50 bis 99,9 Gew.-% und Hinokitiol in einer Menge von 50 bis 0,1 Gew.-% verwendet wird.

36. Mittel gegen Bakterien und/oder Pilze, das eine Zinkverbindung und mindestens ein Mitglied, ausgewählt aus der Gruppe, bestehend aus Hinokitiol und seinen Salzen, umfaßt, unter der Voraussetzung, daß, die Zinkverbindung weder Zinkdipicolinat, ein Zinksalz der Edetinsäure, Zinkphenolsulfonat noch Zinkparaphenolsulfonat ist.

37. Mittel gegen Bakterien und/oder Pilze gemäß Anspruch 36, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

38. Mittel gegen Bakterien und/oder Pilze gemäß Anspruch 37, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 10 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

39. Mittel gegen Bakterien und/oder Pilze gemäß Anspruch 36, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zink-Glycerin-Komplex, Zink-Bishistidin-Komplex, Zink-3,4-dihydroxybenzoesäure-Komplex, Zinknicotinat und Zink-Nicotinamid-Komplex und das mindestens eine Mitglied, ausgewählt aus der Gruppe, bestehend aus Hinokitiol und seinen Salzen, mindestens ein Mitglied ist, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols.

40. Mittel gegen Bakterien und/oder Pilze gemäß Anspruch 37, worin das Mittel Zinkoxid und mindestens ein Mitglied ausgewählt aus der Gruppe, bestehend aus Hinokitiol und seinen Salzen, umfaßt, wobei das Mittel Zinkoxid und das mindestens eine Mitglied, ausgewählt aus der Gruppe, bestehend aus Hinokitiol oder seinen Salzen, in einer Gesamtmenge von 0,001 bis 20 Gew.-% enthält, und, bezogen auf die Gesamtmenge an Zinkoxid und dem mindestens einem Mitglied, ausgewählt aus der Gruppe, bestehend aus Hinokitiol und seinen Salzen, Zinkoxid in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

41. Mittel gegen Bakterien und/oder Pilze gemäß Anspruch 40, worin das Mittel Zinkoxid und mindestens ein Mitglied, ausgewählt aus der Gruppe, bestehend aus Hinokitiol und seinen Salzen, umfaßt, wobei das Mittel Zinkoxid und das mindestens eine Mitglied, ausgewählt aus der Gruppe, bestehend aus Hinokitiol oder seinen Salzen, in einer Gesamtmenge von 0,01 bis 15 Gew.-% enthält, und, bezogen auf die Gesamtmenge an Zinkoxid und dem mindestens einen Mitglied ausgewählt aus der Gruppe, bestehend aus Hinokitiol und seinen Salzen, Zinkoxid in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

42. Mittel gegen Bakterien und/oder Pilze gemäß Anspruch 36, worin das Mittel Zinkpyrithion und mindestens ein Mitglied, ausgewählt aus der Gruppe, bestehend aus Hinokitiol und seinen Salzen, umfaßt, wobei das Mittel Zinkpyrithion und das mindestens eine Mitglied, ausgewählt aus der Gruppe, bestehend aus Hinokitiol oder seinen Salzen, in einer Gesamtmenge von 0,001 bis 20 Gew.-% enthält, und, bezogen auf die Gesamtmenge an Zinkpyrithion und dem mindestens einem Mitglied, ausgewählt aus der Gruppe, bestehend aus Hinokitiol und seinen Salzen, Zinkpyrithion in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

43. Mittel gegen Bakterien und/oder Pilze gemäß Anspruch 42, worin das Mittel Zinkpyrithion und mindestens ein Mitglied, ausgewählt aus der Gruppe, bestehend aus Hinokitiol und seinen Salzen, umfaßt, wobei das Mittel Zinkpyrithion und das mindestens eine Mitglied, ausgewählt aus der Gruppe, bestehend aus Hinokitiol oder seinen Salzen, in einer Gesamtmenge von 0,01 bis 15 Gew.-% enthält, und, bezogen auf die Gesamtmenge an Zinkpyrithion und dem mindestens einem Mitglied, ausgewählt aus der Gruppe, bestehend aus Hinokitiol und seinen Salzen, Zinkpyrithion in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

44. Mittel gegen Bakterien und/oder Pilze gemäß Anspruch 36, worin das Mittel Zinkmyristat und mindestens ein Mitglied, ausgewählt aus der Gruppe, bestehend aus Hinokitiol und seinen Salzen, umfaßt, wobei das Mittel Zinkmyristat und das mindestens eine Mitglied, ausgewählt aus der Gruppe, bestehend aus Hinokitiol oder seinen Salzen, in einer Gesamtmenge von 0,001 bis 20 Gew.-% enthält, und, bezogen auf die Gesamtmenge an Zinkmyristat und dem mindestens einem Mitglied, ausgewählt aus der Gruppe, bestehend aus Hinokitiol und seinen Salzen, Zinkmyristat in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

45. Mittel gegen Bakterien und/oder Pilze gemäß Anspruch 44, worin das Mittel Zinkmyristat und mindestens ein Mitglied, ausgewählt aus der Gruppe, bestehend aus Hinokitiol und seinen Salzen, umfaßt, wobei das Mittel Zinkmyristat und das mindestens eine Mitglied, ausgewählt aus der Gruppe, bestehend aus Hinokitiol oder seinen Salzen, in einer Gesamtmenge von 0,01 bis 15 Gew.-% enthält, und, bezogen auf die Gesamtmenge an Zinkmyristat und dem mindestens einem Mitglied, ausgewählt aus der Gruppe, bestehend aus Hinokitiol und seinen Salzen, Zinkmyristat in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

46. Kosmetikum, das eine Zinkverbindung und mindestens ein Mitglied, ausgewählt aus der Gruppe, bestehend aus Hinokitiol und seinen Salzen, enthält, unter der Voraussetzung, daß die Zinkverbindung nicht Zinkparaphenolsulfonat, Zinkphenolsulfonat, Zinkdipicolinat oder ein Zinksalz der Edetinsäure darstellt.

47. Kosmetikum gemäß Anspruch 46, worin die Zinkverbindung und das mindestens eine Mitglied, ausgewählt aus der Gruppe, bestehend aus Hinokitiol und seinen Salzen als Antischuppenmittel, Bakterizid und/oder Konservierungsmittel inkorporiert werden.

48. Kosmetikum gemäß Anspruch 47, worin das Verhältnis der Zinkverbindung (A) und des mindestens einen Mitglieds (B), ausgewählt aus der Gruppe bestehend aus Hinokitiol und seinen Salzen, A:B = 10 bis 99,95:90 bis 0,05 per Gewicht beträgt.

49. Kosmetikum gemäß Anspruch 48, worin das Verhältnis der Zinkverbindung (A) und des mindestens einen Mitglieds (B), ausgewählt aus der Gruppe, bestehend aus Hinokitiol und seinen Salzen, A:B = 50 bis 99,9:50 bis 0,1 per Gewicht beträgt.

50. Kosmetikum gemäß Anspruch 47, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

51. Kosmetikum gemäß Anspruch 50, worin, bezogen auf die Gesamtmenge der Zinkverbindung und des mindestens einen Mitglieds, ausgewählt aus der Gruppe, bestehend aus Hinokitiol und seinen Salzen, die Zinkverbindung in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

52. Kosmetikum gemäß irgendeinem der Ansprüche 47 bis 51, worin die Zinkverbindung mindestens ein Mitglied, ausgewählt aus der Gruppe, bestehend aus Zinkoxid, Zinkchlorid, Zinknitrat, Zinksulfat, Zinkphosphat, Zinkaluminat, Zinkfluorid, Zinkjodid, Zinkhydroxid, Zinkcarbonat, Zinkchromat, Zinkbenzoat, Zinkacetat, Zinkparaaminobenzoat, Zinkparadimethylaminobenzoat, Zinkparamethoxycinnamat, Zinklactat, Zink-2-mercaptopyridin-N-oxid-Komplex, Zinkgluconat, Zinkpicrat, Zinkcitrat, Zinkaspartat, Zinknaphthenat, Zinksalicylat, Zinksebacat, Natriumzinktripolyphosphat, Zinkstearat, Zinkcaprat, Zinklaurat, Zinkmyristat, Zinkpalmitat, Zinkoleat, Zinkpolyphosphonat, Zinkchondroitinsulfat, Zinkundecylenat, Zinkascorbat, Zinkpyrithion, Hinokitiolatozink, Zink-Glycerin-Komplex, Zink-Bishistidin-Komplex, Zink-3,4-dihydroxybenzoesäure-Komplex, Zinknicotinat und Zink-Nicotinamid-Komplex ist.

53. Kosmetikum gemäß Anspruch 52, worin das mindestens eine Mitglied, ausgewählt aus der Gruppe, bestehend aus Hinokitiol und seinen Salzen, mindestens ein Mitglied, ausgewählt aus der Gruppe, bestehend aus Hinokitiol und einem Natriumsalz, Kaliumsalz, Magnesiumsalz, Kupfersalz, Zinksalz, Diethanolaminsalz, 2-Amino-2-ethyl-1,3-propandiolsalz, Triethanolaminsalz, Morpholinsalz, Piperazinsalz, Piperidinsalz, Ammoniumsalz, Argininsalz, Lysinsalz und Histidinsalz des Hinokitiols ist.

54. Kosmetikum gemäß Anspruch 47, worin, bezogen auf die Gesamtmenge der Zinkverbindung (a) und dem mindestens einen Mitglied (b), ausgewählt aus der Gruppe, bestehend aus Hinokitiol und seinen Salzen, die Zinkverbindung (a) in einer Menge von 50 bis 99,9 Gew.-% und das mindestens eine Mitglied (b), ausgewählt aus der Gruppe, bestehend aus Hinokitiol und seinen Salzen, in einer Menge von 50 bis 0,1 Gew.-% verwendet wird (werden), wobei die Zinkverbindung (a) mindestens ein Mitglied, ausgewählt aus der Gruppe, bestehend aus Zinkoxid, Zinkacetat, Zinkpyrithion, Hinokitiolatozink und Zinkchlorid und die Verbindung (b) mindestens ein Mitglied, ausgewählt aus der Gruppe bestehend aus Hinokitiol und Hinokitiol-Natriumsalz ist.

55. Kosmetikum gemäß Anspruch 46, worin das Kosmetikum Zinkoxid und mindestens ein Mitglied, ausgewählt aus der Gruppe, bestehend aus Hinokitiol und seinen Salzen, umfaßt, wobei das Kosmetikum Zinkoxid und das mindestens eine Mitglied, ausgewählt aus der Gruppe, bestehend aus Hinokitiol oder seinen Salzen, in einer Gesamtmenge von 0,0001 bis 99,9 Gew.-% enthält, und, bezogen auf die Gesamtmenge an Zinkoxid und dem mindestens einen Mitglied, ausgewählt aus der Gruppe, bestehend aus Hinokitiol und seinen Salzen, Zinkoxid in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

56. Kosmetikum gemäß Anspruch 55, worin das Kosmetikum Zinkoxid und mindestens ein Mitglied, ausgewählt aus der Gruppe, bestehend aus Hinokitiol und seinen Salzen, umfaßt, wobei das Kosmetikum Zinkoxid und das mindestens eine Mitglied, ausgewählt aus der Gruppe, bestehend aus Hinokitiol oder seinen Salzen, in einer Gesamtmenge von 0,001 bis 30 Gew.-% enthält, und, bezogen auf die Gesamtmenge an Zinkoxid und dem mindestens einen Mitglied, ausgewählt aus der Gruppe, bestehend aus Hinokitiol und seinen Salzen, Zinkoxid in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

57. Kosmetikum gemäß Anspruch 46, worin das Kosmetikum Zinkpyrithion und mindestens ein Mitglied, ausgewählt aus der Gruppe, bestehend aus Hinokitiol und seinen Salzen, umfaßt, wobei das Kosmetikum Zinkpyrithion und das mindestens eine Mitglied, ausgewählt aus der Gruppe, bestehend aus Hinokitiol oder seinen Salzen, in einer Gesamtmenge von 0,0001 bis 99,9 Gew.-% enthält, und, bezogen auf die Gesamtmenge an Zinkpyrithion und dem mindestens einen Mitglied, ausgewählt aus der Gruppe, bestehend aus Hinokitiol und seinen Salzen, Zinkpyrithion in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

58. Kosmetikum gemäß Anspruch 57, worin das Kosmetikum Zinkpyrithion und mindestens ein Mitglied, ausgewählt aus der Gruppe, bestehend aus Hinokitiol und seinen Salzen, umfaßt, wobei das Kosmetikum Zinkpyrithion und das mindestens eine Mitglied ausgewählt aus der Gruppe, bestehend aus Hinokitiol oder seinen Salzen, in einer Gesamtmenge von 0,001 bis 30 Gew.-% enthält, und, bezogen auf die Gesamtmenge an Zinkpyrithion und dem mindestens einen Mitglied, ausgewählt aus der Gruppe, bestehend aus Hinokitiol und seinen Salzen, Zinkpyrithion in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

59. Kosmetikum gemäß Anspruch 46, worin das Kosmetikum Zinkmyristat und mindestens ein Mitglied, ausgewählt aus der Gruppe, bestehend aus Hinokitiol und seinen Salzen, umfaßt, wobei das Kosmetikum Zinkmyristat und das mindestens eine Mitglied, ausgewählt aus der Gruppe, bestehend aus Hinokitiol oder seinen Salzen, in einer Gesamtmenge von 0,0001 bis 99,9 Gew.-% enthält, und, bezogen auf die Gesamtmenge an Zinkmyristat und dem mindestens einen Mitglied, ausgewählt aus der Gruppe, bestehend aus Hinokitiol und seinen Salzen, Zinkmyristat in einer Menge von 10 bis 99,95 Gew.-% und das mindestens eine Mitglied, ausgewählt aus der Gruppe, bestehend aus Hinokitiol und seinen Salzen, in einer Menge von 90 bis 0,05 Gew.-% verwendet wird (werden).

60. Kosmetikum gemäß Anspruch 59, worin das Kosmetikum Zinkmyristat und mindestens ein Mitglied, ausgewählt aus der Gruppe, bestehend aus Hinokitiol und seinen Salzen, umfaßt, wobei das Kosmetikum Zinkmyristat und das mindestens eine Mitglied, ausgewählt aus der Gruppe, bestehend aus Hinokitiol oder seinen Salzen, in einer Gesamtmenge von 0,001 bis 30 Gew.-% enthält, und, bezogen auf die Gesamtmenge an Zinkmyristat und dem mindestens einen Mitglied, ausgewählt aus der Gruppe, bestehend aus Hinokitiol und seinen Salzen, Zinkmyristat in einer Menge von 50 bis 99,9 Gew.-% und das mindestens eine Mitglied, ausgewählt aus der Gruppe, bestehend aus Hinokitiol und seinen Salzen, in einer Menge von 50 bis 0,1 Gew.-% verwendet wird (werden).

61. Kosmetikum gemäß Anspruch 54, worin, bezogen auf die Gesamtmenge des Zinkoxids und des Hinokitiols, das Zinkoxid in einer Menge von 50 bis 99,9 Gew.-% und das Hinokitiol in einer Menge von 50 bis 0,1 Gew.-% verwendet wird.

62. Verfahren zur Herstellung eines Medikaments, das bei der Behandlung einer durch Bakterien oder Pilze verursachten Infektionskrankheit wirksam ist, umfassend den Schritt des Inkorporierens einer Zinkverbindung und mindestens eines Mitglieds, ausgewählt aus der Gruppe, bestehend aus Hinokitiol und seinen Salzen, zu einem pharmazeutisch verträglichen Verdünnungsmittel oder Vehikel, unter der Voraussetzung, daß die Zinkverbindung kein Zinksalz der Edetinsäure ist.

## Revendications

1. Utilisation d'un composé du zinc et d'au moins un composé choisi dans le groupe consistant en hinokitiol et sels de celui-ci pour la préparation d'un médicament efficace dans le traitement d'une maladie infectieuse provoquée par des bactéries ou des champignons.

2. Utilisation suivant la revendication 1, dans laquelle la dite maladie infectieuse est provoquée par des bactéries.

3. Utilisation suivant les revendications 1 ou 2, dans laquelle le rapport du dit composé du zinc (A) et du dit composé (B) au moins présent choisi dans le groupe consistant en hinokitiol et sels de celui-ci est A:B = 10 à 99,95 : 90 à 0,05 en poids.

4. Utilisation suivant la revendication 3, dans laquelle le rapport du dit composé du zinc (A) et du dit composé (B) au moins présent choisi dans le groupe consistant en hinokitiol et sels de celui-ci est A:B = 50 à 99,9 : 50 à 0,1 en poids.

5. Utilisation suivant les revendications 1 ou 2, dans laquelle, par rapport à la quantité totale du dit composé du zinc et du dit composé au moins présent choisi dans le groupe consistant en hinokitiol et sels de celui-ci, le dit composé du zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

6. Utilisation suivant la revendication 5, dans laquelle, par rapport à la quantité totale du dit composé du zinc et du dit composé au moins présent choisi dans le groupe consistant en hinokitiol et sels de celui-ci, le dit composé du zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

7. Utilisation suivant l'une quelconque des revendications 1 à 6, dans laquelle le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraphénolsulfonate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, phénolsulfonate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, dipicolinate de zinc, complexe de zinc-glycérol, complexe de zinc-bishistidine, complexe de zinc-acide 3,4-dihydroxybenzoïque, nicotinate de zinc et complexe de zinc-nicotinamide.

8. Utilisation suivant la revendication 7, dans laquelle le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

9. Utilisation suivant la revendication 8, dans laquelle le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, complexe de zinc-glycérol, complexe de zinc-bishistidine, complexe de zinc-acide 3,4-dihydroxybenzoïque, nicotinate de zinc et complexe de zinc-nicotinamide, et le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

10. Utilisation suivant les revendications 1 ou 2, dans laquelle, par rapport à la quantité totale du dit composé du zinc (A) et du dit composé (B) au moins présent choisi dans le groupe consistant en hinokitiol et sels de celui-ci, le dit composé du zinc (A) est utilisé en une quantité de 50 à 99,9% en poids et le dit composé (B) au moins présent choisi dans le groupe consistant en hinokitiol et sels de celui-ci est utilisé en une quantité de 50 à 0,1% en poids, le dit composé du zinc (A) étant au moins un composé choisi dans le groupe consistant en oxyde de zinc, acétate de zinc, zinc pyrithione, hinokitiolato zinc, chlorure de zinc et dipicolinate de zinc, et le dit composant (B) étant au moins un composé choisi dans le groupe consistant en hinokitiol et sel de sodium d'hinokitiol.

11. Utilisation suivant la revendication 10, dans laquelle, par rapport à la quantité totale du dit composé du zinc (A) et du dit composé (B) au moins présent choisi dans le groupe consistant en hinokitiol et sels de celui-ci, le dit composé du zinc (A) est utilisé en une quantité de 50 à 99,9% en poids et le dit composé (B) au moins présent choisi dans le groupe consistant en hinokitiol et sels de celui-ci est utilisé en une quantité de 50 à 0,1% en poids, le dit composé du zonc (A) étant au moins un composé choisi dans le groupe consistant en oxyde de zinc, acétate de zinc, zinc pyrithione, hinokitiolato zinc et chlorure de zinc, et le dit composant (B) étant au moins un composé choisi dans le groupe consistant en hinokitiol et sel de sodium d'hinokitiol.

12. Utilisation suivant les revendications 1 ou 2, dans laquelle le dit composé du zinc est l'oxyde de zinc, et par rapport à la quantité totale d'oxyde de zinc et du dit composé au moins présent choisi dans le groupe consistant en hinokitiol et sels de celui-ci, l'oxyde de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

13. Utilisation suivant la revendication 12, dans laquelle le dit composé du zinc est l'oxyde de zinc, et par rapport à la quantité totale d'oxyde de zinc et du dit composé au moins présent choisi dans le groupe consistant en hinokitiol et sels de celui-ci, l'oxyde de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

14. Utilisation suivant les revendications 1 ou 2, dans laquelle le dit composé du zinc est la zinc pyrithione, et par rapport à la quantité totale de zinc pyrithione et du dit composé au moins présent choisi dans le groupe consistant en hinokitiol et sels de celui-ci, la zinc pyrithione est utilisée en une quantité de 10 à 99,95% en poids et le dit composé au moins présent choisi dans le groupe consistant en hinokitiol et sels de celui-ci est utilisé en une quantité de 90 à 0,05% en poids.

15. Utilisation suivant la revendication 14, dans laquelle le dit composé du zinc est la zinc pyrithione, et par rapport à la quantité totale de zinc pyrithione et du dit composé au moins présent choisi dans le groupe consistant en hinokitiol et sels de celui-ci, la zinc pyrithione est utilisée en une quantité de 50 à 99,9% en poids et le dit composé au moins présent choisi dans le groupe consistant en hinokitiol et sels de celui-ci est utilisé en une quantité de 50 à 0,1% en poids.

16. Utilisation suivant les revendications 1 ou 2, dans laquelle le dit composé du zinc est le myristate de zinc, et par rapport à la quantité totale de myristate de zinc et du dit composé au moins présent choisi dans le groupe consistant en hinokitiol et sels de celui-ci, le myristate de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

17. Utilisation suivant la revendication 16, dans laquelle le dit composé du zinc est le myristate de zinc, et par rapport à la quantité totale de myristate de zinc et du dit composé au moins présent choisi dans le groupe consistant en hinokitiol et sels de celui-ci, le myristate de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

18. Utilisation suivant la revendication 13, dans laquelle, par rapport à la quantité totale d'oxyde de zinc et d'hinokitiol, l'oxyde de zinc est utilisé en une quantité de 50 à 99,9% en poids et l'hinokitiol est utilisé en une quantité de 50 à 0,1% en poids.

19. Procédé pour la conservation d'un cosmétique qui comprend l'introduction dans le dit cosmétique d'un composé du zinc et d'au moins un composé choisi dans le groupe consistant en hinokitiol et sels de celui-ci.

20. Procédé suivant la revendication 19, dans lequel le rapport du dit composé du zinc (A) et du dit composé (B) au moins présent choisi dans le groupe consistant en hinokitiolet sels de celui-ci est A : B = 10 à 99,95 : 90 à 0,05. en poids.

21. Procédé suivant la revendication 20, dans lequel le rapport du dit composé du zinc (A) et du dit composé (B) au moins présent choisi dans le groupe consistant en hinokitiol et sels de celui-ci est A:B = 50 à 99,9 : 50 à 0,1 en poids.

22. Procédé suivant la revendication 19, dans lequel, par rapport à la quantité totale du dit composé du zinc et du dit composé au moins présent choisi dans le groupe consistant en hinokitiol et sels de celui-ci, le dit composé du zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

23. Procédé suivant la revendication 22, dans lequel, par rapport à la quantité totale du dit composé du zinc et du dit composé au moins présent choisi dans le groupe consistant en hinokitiol et sels de celui-ci, le dit composé du zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

24. Procédé suivant l'une quelconque des revendications 19 à 23, dans lequel le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraphénolsulfonate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, phénolsulfonate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, dipicolinate de zinc, complexe de zinc-glycérol, complexe de zinc-bishistidine, complexe de zinc-acide 3,4-dihydroxybenzoïque, nicotinate de zinc et complexe de zinc-nicotinamide.

25. Procédé suivant la revendication 24, dans lequel le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

26. Procédé suivant la revendication 25, dans lequel le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, complexe de zinc-glycérol, complexe de zinc-bishistidine, complexe de zinc-acide 3,4-dihydroxybenzoïque, nicotinate de zinc et complexe de zinc-nicotinamide, et le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

27. Procédé suivant la revendication 19, dans lequel, par rapport à la quantité totale du dit composé du zinc (A) et du dit composé au moins présent choisi dans le groupe consistant en hinokitiol et sels de celui-ci, le dit composé du zinc (A) est utilisé en une quantité de 50 à 99,9% en poids et le dit composé au moins présent choisi dans le groupe consistant en hinokitiol et sels de celui-ci est utilisé en une quantité de 50 à 0,1% en poids, le dit composé du zinc (A) étant au moins un composé choisi dans le groupe consistant en oxyde de zinc, acétate de zinc, zinc pyrithione, hinokitiolato zinc, chlorure de zinc et dipicolinate de zinc, et le dit composant (B) étant au moins un composé choisi dans le groupe consistant en hinokitiol et sel de sodium d'hinokitiol.

28. Procédé suivant la revendication 27, dans lequel, par rapport à la quantité totale du dit composé du zinc (A) et du dit composé au moins présent choisi dans le groupe consistant en hinokitiol et sels de celui-ci, le dit composé du zinc (A) est utilisé en une quantité de 50 à 99,9% en poids et le dit composé au moins présent choisi dans le groupe consistant en hinokitiol et sels de celui-ci est utilisé en une quantité de 50 à 0,1% en poids, le dit composé du zinc (A) étant au moins un composé choisi dans le groupe consistant en oxyde de zinc, acétate de zinc, zinc pyrithione, hinokitiolato zinc et chlorure de zinc, et le dit composant (B) étant au moins un composé choisi dans le groupe consistant en hinokitiol et sel de sodium d'hinokitiol.

29. Procédé suivant la revendication 19, dans lequel le dit composé de zinc est l'oxyde de zinc, et par rapport à la quantité totale d'oxyde de zinc et du dit composé au moins présent choisi dans le groupe consistant en hinokitiol et sels de celui-ci, l'oxyde de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

30. Procédé suivant la revendication 29, dans lequel le dit composé du zinc est l'oxyde de zinc, et par rapport à la quantité totale d'oxyde de zinc et du dit composé au moins présent choisi dans le groupe consistant en hinokitiol et sels de celui-ci, l'oxyde de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

31. Procédé suivant la revendication 19, dans lequel le dit composé du zinc est la zinc pyrithione, et par rapport à la quantité totale de zinc pyrithione et du dit composé au moins présent choisi dans le groupe consistant en hinokitiol et sels de celui-ci, la zinc pyrithione est utilisée en une quantité de 10 à 99,95% en poids et le dit composé au moins présent choisi dans le groupe consistant en hinokitiol et sels de celui-ci est utilisé en une quantité de 90 à 0,05% en poids.

32. Procédé suivant la revendication 31, dans lequel le dit composé du zinc est la zinc pyrithione, et par rapport à la quantité totale de zinc pyrithione et du dit composé au moins présent choisi dans le groupe consistant en hinokitiol et sels de celui-ci, la zinc pyrithione est utilisée en une quantité de 50 à 99,9% en poids et le dit composé au moins présent choisi dans le groupe consistant en hinokitiol et sels de celui-ci est utilisé en une quantité de 50 à 0,1% en poids.

33. Procédé suivant la revendication 19, dans lequel le dit composé du zinc est le myristate de zinc, et par rapport à la quantité totale de myristate de zinc et du dit composé au moins présent choisi dans le groupe consistant en hinokitiol et sels de celui-ci, le myristate de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

34. Procédé suivant la revendication 33, dans lequel le dit composé du zinc est le myristate de zinc, et par rapport à la quantité totale de myristate de zinc et du dit composé au moins présent choisi dans le groupe consistant en hinokitiol et sels de celui-ci, le myristate de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

35. Procédé suivant la revendication 30, dans lequel, par rapport à la quantité totale d'oxyde de zinc et d'hinokitiol, l'oxyde de zinc est utilisé en une quantité de 50 à 99,9% en poids et l'hinokitiol est utilisé en une quantité de 50 à 0,1% en poids.

36. Agent antibactérien et/ou antifongique qui comprend un composé du zinc et au moins un composé choisi dans le groupe consistant en hinokitiol et sels de celui-ci à condition que le composé du zinc ne soit ni le dipicolinate de zinc, ni un sel de zinc d'acide édétique, ni le phénol sulfonate de zinc, ni le paraphénolsulfonate de zinc.

37. Agent antibactérien et/ou antifongique suivant la revendication 36, dans lequel, par rapport à la quantité totale du dit composé du zinc et du dit composé au moins présent choisi dans le groupe consistant en hinokitiol et sels de celui-ci, le dit composé du zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

38. Agent antibactérien et/ou antifongique suivant la revendication 37, dans lequel, par rapport à la quantité totale du dit composé du zinc et du dit composé au moins présent choisi dans le groupe consistant en hinokitiol et sels de celui-ci, le dit composé du zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

39. Agent antibactérien et/ou antifongique suivant la revendication 36, dans lequel le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, complexe de zinc-glycérol, complexe de zinc-bishistidine, complexe de zinc-acide 3,4-dihydroxybenzoïque, nicotinate de zinc et complexe de zinc-nicotinamide, et le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

40. Agent antibactérien et/ou antifongique suivant la revendication 37, dans lequel le dit agent comprend de l'oxyde de zinc, et le dit composé au moins présent choisi dans le groupe consistant en hinokitiol et sels de celui-ci, le dit agent contient de l'oxyde de zinc et le dit composé au moins présent choisi dans le groupe consistant en hinokitiol ou sels de celui-ci en une quantité totale de 0,001 à 20% en poids et par rapport à la quantité totale d'oxyde de zinc et du dit composé au moins présent choisi dans le groupe consistant en hinokitiol et sels de celui-ci, l'oxyde de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

41. Agent antibactérien et/ou antifongique suivant la revendication 40, dans lequel le dit agent comprend de l'oxyde de zinc, et le dit composé au moins présent choisi dans le groupe consistant en hinokitiol et sels de celui-ci, le dit agent contient de l'oxyde de zinc et le dit composé au moins présent choisi dans le groupe consistant en hinokitiol ou sels de celui-ci en une quantité totale de 0,01 à 15% en poids et par rapport à la quantité totale d'oxyde de zinc et du dit composé au moins présent choisi dans le groupe consistant en hinokitiol et sels de celui-ci, l'oxyde de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

42. Agent antibactérien et/ou antifongique suivant la revendication 36, dans lequel le dit agent comprend de la zinc pyrithione et le dit composé au moins présent choisi dans le groupe consistant en hinokitiol et sels de celui-ci, le dit agent contient la dite zinc pyrithione et le dit composé au moins présent choisi dans le groupe consistant en hinokitiol ou sels de celui-ci en une quantité totale de 0,001 à 20% en poids et par rapport à la quantité totale de la dite zinc pyrithione et du dit composé au moins présent choisi dans le groupe consistant en hinokitiol et sels de celui-ci, la dite zinc pyrithione est utilisée en une quantité de 10 à 99,95% en poids et le dit composé au moins présent choisi dans le groupe consistant en hinokitiol et sels de celui-ci est utilisé en une quantité de 90 à 0,05% en poids.

43. Agent antibactérien et/ou antifongique suivant la revendication 42, dans lequel le dit agent comprend de la Zinc pyrithione et le dit composé au moins présent choisi dans le groupe consistant en hinokitiol et sels de celui-ci, le dit agent contient la dite zinc pyrithione et le dit composé au moins présent choisi dans le groupe consistant en hinokitiol ou sels de celui-ci en une quantité totale de 0,01 à 15% en poids et par rapport à la quantité totale de la dite zinc pyrithione et du dit composé au moins présent choisi dans le groupe consistant en hinokitiol et sels de celui-ci, la dite zinc pyrithione est utilisée en une quantité de 50 à 99,9% en poids et le dit composé au moins présent choisi dans le groupe consistant en hinokitiol ou sels de celui-ci est utilisé en une quantité de 50 à 0,1% en poids.

44. Agent antibactérien et/ou antifongique suivant la revendication 36, dans lequel le dit agent comprend du myristate de zinc et le dit composé au moins présent choisi dans le groupe consistant en hinokitiol et sels de celui-ci, le dit agent contient le dit myristate de zinc et le dit composé au moins présent choisi dans le groupe consistant en hinokitiol ou sels de celui-ci en une quantité totale de 0,001 à 20% en poids et par rapport à la quantité totale du dit myristate de zinc et du dit composé au moins présent choisi dans le groupe consistant en hinokitiol et sels de celui-ci, le dit myristate de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

45. Agent antibactérien et/ou antifongique suivant la revendication 44, dans lequel le dit agent comprend du myristate de zinc et le dit composé au moins présent choisi dans le groupe consistant en hinokitiol et sels de celui-ci, le dit agent contient le dit myristate de zinc et le dit composé au moins présent choisi dans le groupe consistant en hinokitiol ou sels de celui-ci en une quantité totale de 0,01 à 15% en poids et par rapport à la quantité totale du dit myristate de zinc et du dit composé au moins présent choisi dans le groupe consistant en hinokitiol et sels de celui-ci, le dit myristate de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

46. Cosmétique qui contient un composé du zinc et au moins un composé choisi dans le groupe consistant en hinokitiol et sels de celui-ci à condition que le composé du zinc ne soit représenté ni par le paraphénolsulfonate, ni par le phénol sulfonate de zinc, ni par le dipicolinate de zinc, ni par un sel de zinc d'acide édétique.

47. Cosmétique suivant la revendication 46, dans lequel le dit composé du zinc et le dit composé au moins présent choisi dans le groupe choisi dans le groupe consistant en hinokitiol et sels de celui-ci sont incorporés dans ce cosmétique en tant qu'agent anti-pelliculaire, bactéricide et/ou conservateur.

48. Cosmétique suivant la revendication 47, dans lequel le rapport du dit composé du zinc (A) et du dit composé (B) au moins présent choisi dans le groupe consistant en hinokitiol et sels de celui-ci est A : B = 10 à 99,95 : 90 à 0,05 en poids.

49. Cosmétique suivant la revendication 48, dans lequel le rapport du dit composé du zinc (A) et du dit composé (B) au moins présent choisi dans le groupe consistant en hinokitiol et sels de celui-ci est A : B = 50 à 99,9 : 50 à 0,1 en poids.

50. Cosmétique suivant la revendication 47, dans lequel, par rapport à la quantité totale du dit composé du zinc et du dit composé au moins présent choisi dans le groupe consistant en hinokitiol et sels de celui-ci, le dit composé du zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

51. Cosmétique suivant la revendication 50, dans lequel, par rapport à la quantité totale du dit composé du zinc et du dit composé au moins présent choisi dans le groupe consistant en hinokitiol et sels de celui-ci, le dit composé du zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

52. Cosmétique suivant l'une quelconque des revendications 47 à 51, dans lequel le dit composé du zinc est au moins un composé choisi dans le groupe consistant en oxyde de zinc, chlorure de zinc, nitrate de zinc, sulfate de zinc, phosphate de zinc, aluminate de zinc, fluorure de zinc, iodure de zinc, hydroxyde de zinc, carbonate de zinc, chromate de zinc, benzoate de zinc, acétate de zinc, paraaminobenzoate de zinc, paradiméthylaminobenzoate de zinc, paraméthoxycinnamate de zinc, lactate de zinc, complexe de zinc-2-mercaptopyridine N-oxyde, gluconate de zinc, picrate de zinc, citrate de zinc, aspartate de zinc, naphténate de zinc, salicylate de zinc, sébacate de zinc, tripolyphosphate de sodium et de zinc, stéarate de zinc, caprate de zinc, laurate de zinc, myristate de zinc, palmitate de zinc, oléate de zinc, polyphosphonate de zinc, chondroïtinesulfate de zinc, undécylénate de zinc, ascorbate de zinc, zinc pyrithione, hinokitiolato zinc, complexe de zinc-glycérol, complexe de zinc-bishistidine, complexe de zinc-acide 3,4-dihydroxybenzoïque, nicotinate de zinc et complexe de zinc-nicotinamide.

53. Cosmétique suivant la revendication 52, dans lequel le dit composé au moins présent choisi dans le groupe consistant en hinokitiol et sels de celui-ci est au moins un composé choisi dans le groupe consistant en hinokitiol et sel de sodium, sel de potassium, sel de magnésium, sel de cuivre, sel de zinc, sel de diéthanolamine, sel de 2-amino-2-éthyl-1,3-propane-diol, sel de triéthanolamine, sel de morpholine, sel de pipérazine, sel de pipéridine, sel d'ammonium, sel d'arginine, sel de lysine et sel d'histidine d'hinokitiol.

54. Cosmétique suivant la revendication 47, dans lequel, par rapport à la quantité totale du dit composé du zinc (A) et du dit composé (B) au moins présent choisi dans le groupe consistant en hinokitiol et sels de celui-ci, le dit composé du zinc (A) est utilisé en une quantité de 50 à 99,9% en poids et le dit composé (B) au moins présent choisi dans le groupe consistant en hinokitiol et sels de celui-ci est utilisé en une quantité de 50 à 0,1% en poids, le dit composé du zinc (A) étant au moins un composé choisi dans le groupe consistant en oxyde de zinc, acétate de zinc, zinc pyrithione, hinokitiolato zinc et chlorure de zinc, et le dit composant (B) étant au moins un composé choisi dans le groupe consistant en hinokitiol et sel de sodium d'hinokitiol.

55. Cosmétique suivant la revendication 46, dans lequel le dit cosmétique comprend de l'oxyde de zinc et le dit composé au moins présent choisi dans le groupe consistant en hinokitiol et sels de celui-ci, le dit cosmétique contient de l'oxyde de zinc et le dit composé au moins présent choisi dans le groupe consistant en hinokitiol ou sels de celui-ci en une quantité totale de 0,0001 à 99,9% en poids et par rapport à la quantité totale d'oxyde de zinc et du dit composé au moins présent choisi dans le groupe consistant en hinokitiol et sels de celui-ci, l'oxyde de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

56. Cosmétique suivant la revendication 55, dans lequel le dit cosmétique comprend de l'oxyde de zinc et le dit composé au moins présent choisi dans le groupe consistant en hinokitiol et sels de celui-ci, le dit cosmétique contient de l'oxyde de zinc et le dit composé au moins présent choisi dans le groupe consistant en hinokitiol ou sels de celui-ci en une quantité totale de 0,001 à 30% en poids et par rapport à la quantité totale d'oxyde de zinc et du dit composé au moins présent choisi dans le groupe consistant en hinokitiol et sels de celui-ci, l'oxyde de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

57. Cosmétique suivant la revendication 46, dans lequel le dit cosmétique comprend de la zinc pyrithione et le dit composé au moins présent choisi dans le groupe consistant en hinokitiol et sels de celui-ci, le dit cosmétique contient la dite zinc pyrithione et le dit composé au moins présent choisi dans le groupe consistant en hinokitiol ou sels de celui-ci en une quantité totale de 0,0001 à 99,9% en poids et par rapport à la quantité totale de la dite zinc pyrithione et du dit composé au moins présent choisi dans le groupe consistant en hinokitiol et sels de celui-ci, la dite zinc pyrithione est utilisée en une quantité de 10 à 99,95% en poids et le dit composé au moins présent choisi dans le groupe consistant en hinokitiol et sels de celui-ci est utilisé en une quantité de 90 à 0,05% en poids.

58. Cosmétique suivant la revendication 57, dans lequel le dit cosmétique comprend de la zinc pyrithione et le dit composé au moins présent choisi dans le groupe consistant en hinokitiol et sels de celui-ci, le dit cosmétique contient la dite zinc pyrithione et le dit composé au moins présent choisi dans le groupe consistant en hinokitiol ou sels de celui-ci en une quantité totale de 0,001 à 30% en poids et par rapport à la quantité totale de la dite zinc pyrithione et du dit composé au moins présent choisi dans le groupe consistant en hinokitiol et sels de celui-ci, la dite zinc pyrithione est utilisée en une quantité de 50 à 99,9% en poids et le dit composé au moins présent choisi dans le groupe consistant en hinokitiol et sels de celui-ci est utilisé en une quantité de 50 à 0,1% en poids.

59. Cosmétique suivant la revendication 46, dans lequel le dit cosmétique comprend du myristate de zinc et le dit composé au moins présent choisi dans le groupe consistant en hinokitiol et sels de celui-ci, le dit cosmétique contient le dit myristate de zinc et le dit composé au moins présent choisi dans le groupe consistant en hinokitiol ou sels de celui-ci en une quantité totale de 0,0001 à 99,9% en poids et par rapport à la quantité totale du dit myristate de zinc et du dit composé au moins présent choisi dans le groupe consistant en hinokitiol et sels de celui-ci, le dit myristate de zinc est utilisé en une quantité de 10 à 99,95% en poids et le dit composé au moins présent choisi dans le groupe consistant en hinokitiol et sels de celui-ci est utilisé en une quantité de 90 à 0,05% en poids.

60. Cosmétique suivant la revendication 59, dans lequel le dit cosmétique comprend du myristate de zinc et le dit composé au moins présent choisi dans le groupe consistant en hinokitiol et sels de celui-ci, le dit cosmétique contient le dit myristate de zinc et le dit composé au moins présent choisi dans le groupe consistant en hinokitiol ou sels de celui-ci en une quantité totale de 0,001 à 30% en poids et par rapport à la quantité totale du dit myristate de zinc et du dit composé au moins présent choisi dans le groupe consistant en hinokitiol et sels de celui-ci, le dit myristate de zinc est utilisé en une quantité de 50 à 99,9% en poids et le dit composé au moins présent choisi dans le groupe consistant en hinokitiol et sels de celui-ci est utilisé en une quantité de 50 à 0,1% en poids.

61. Cosmétique suivant la revendication 54, dans lequel, par rapport à la quantité totale d'oxyde de zinc et d'hinokitiol, l'oxyde de zinc est utilisé en une quantité de 50 à 99,9% en poids et l'hinokitiol en une quantité de 50 à 0,1% en poids.

62. Procédé pour préparer un médicament efficace dans le traitement d'une maladie infectieuse provoquée par des bactéries ou des champignons comprenant l'étape d'incorporation d'un composé du zinc et d'au moins un composé choisi dans le groupe consistant en hinokitiol et sels de celui-ci dans un diluant ou excipient acceptable du point de vue pharmaceutique, à condition que le composé du zinc ne soit pas un sel de zinc d'acide édétique.
